# EUROPEAN PATENT SPECIFICATION

(11) **EP 3 140 415 B1**
(45) Date of publication and mention of the grant of the patent: **28.09.2022**
(21) Application number: 15789521.0
(22) Date of filing: 05.05.2015
(51) Int. Cl.: A01K 67/033, C07K 14/705, G01N 33/68

(54) **EXPRESSION OF VOLTAGE-GATED ION CHANNELS IN CILIATES**
EXPRESSION VON SPANNUNGSABHÄNGIGEN IONENKANÄLEN IN CILIATEN
EXPRESSION DE CANAUX IONIQUES VOLTAGE-DÉPENDANTS CHEZ LES CILIÉS

(30) Priority: 05.05.2014 US 201461988647 P; 24.06.2014 US 201462016377 P
(43) Date of publication of application: 15.03.2017
(73) Proprietor: Tetragenetics, Inc., Cambridge, MA 02140 (US)
(72) Inventor: COLUSSI, Paul, Gloucester, MA 01930 (US); PAPOYAN, Ashot, Arlington, MA 02474 (US); BISHARYAN, Yelena, Arlingotn, MA 02474 (US); BEDNENKO, Janna, Woburn, MA 01801 (US); CLARK, Theodore, G., Ithaca, NY 14850 (US)
(74) Representative: HGF
(86) International application number: PCT/US2015/029290
(87) International publication number: WO 2015/171643

(56) References cited:
- WO-A1-00/49048
- WO-A1-01/14403
- WO-A1-2010/108183
- WO-A1-2011/119498
- US-A1- 2005 032 165
- US-A1- 2005 202 539
- US-A1- 2005 202 539
- US-A1- 2009 175 784
- GAERTIG J. ET AL.: "Suface display of a parasite antigen in the ciliate Tetrahymena thermophila", NATURE BIOTECHNOL., vol. 17, no. 5, 17 May 1999 (1999-05-17), pages 462-465, XP002141865,
- FORMIGARI A. ET AL.: "Functional Characterization of the 52-upstream Region of MTT5 Metallothionein Gene from Tetrahymena thermophila", PROTIST, vol. 161, no. 1, January 2010 (2010-01), pages 71-77, XP026817712,
- SANTARELLI V.P. ET AL.: "A cation-[pi] interaction discriminates among sodium channels that are either sensitive or resistant to tetrodotoxin block", J. BIOL. CHEM., vol. 282, no. 11, 19 January 2007 (2007-01-19), pages 8044-8051, XP055418688,
- JAVAUX ET AL.: 'Recognizing and interpreting the fossils of early eukaryotes' ORIGINS OF LIFE AND EVOLUTION OF THE BIOSPHERE vol. 33, no. 1, February 2003, pages 75 - 94, XP055360435
- BENNETT ET AL.: 'Isoform-specific effects of sialic acid on voltage-dependent Na+ channel gating: functional sialic acids are localized to the S5-S6 loop of domain I' JOURNAL OF PHYSIOLOGY vol. 538, no. 3, 2002, pages 675 - 690, XP055360441
- ASHMORE J.K. ET AL.: "Ionic basis of membrane potential in outer hair cells of guinea pig cocklea", NATURE, vol. 322, no. 6077, 1986, pages 368-371,
- RUDBERG P. ET AL.: "Bistable membrane potential of the ciliate Coleps hirtus", J. EXP. BIOL., vol. 203, no. 4, February 2000 (2000-02), pages 757-764,
- ECHEVARRIA M.L. ET AL.: "Feast or flee: bioelectrical regulation of feeding and predator evasion behaviors in the planktonic alveolate Favella sp. (Spirotrichia)", J. EXP. BIOL., vol. 219, no. 3, February 2016 (2016-02), pages 445-456,

## Description

### BACKGROUND OF THE INVENTION

### Field of the Invention

The invention relates to the expression of voltage-gated ion channels in ciliates, such as *Tetrahymena.* In particular, the invention provides for high levels of expression of voltage-gated ion channels which are correctly folded and embedded in the plasma membrane at high density, thereby providing for membrane preparations with high densities of such voltage-gated ion channels.

### Description of the Related Art

Voltage gated ion channels (VGIC) are membrane proteins that regulate a variety of physiological processes by conducting ions across biological membranes. The human genome encodes 143 VGIC family members making it the third largest superfamily of signal-transducing proteins after protein kinases and G-protein coupled receptors (Yu and Catterall (2004)).

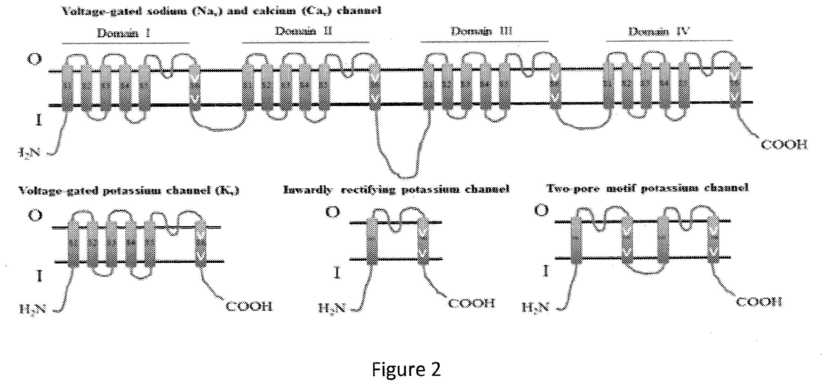

VGICs are commonly classified by selectivity for the ion (K⁺, Na⁺, Ca²⁺) that is primarily conducted across the membrane. However, regardless of ion selectivity, VGIC family members share a common structural theme featuring a principal pore-forming alpha (α) subunit. There are four variations of this structural theme: two structural variations are based on domains comprising six transmembrane (TM) segments and two structural variations are based on domains comprising two transmembrane segments (Figure 2). Of the six TM segment channels, sodium (Naᵥ) and calcium (Caᵥ) α subunits are comprised of four homologous domains (I-IV) with each domain consisting of six transmembrane helices termed S1-S6 with a further membrane reentrant loop between the S5 and S6 helices. The four homologous domains surround a central pore created by the S5 and S6 transmembrane segments and the membrane reentrant pore loop between them. The pore forming S5 and S6 structural elements also comprise the ion selectivity filter of the ion channel. The S1-S4 helices of each α subunit domain confer voltage dependent opening of the ion channel. The S4 segment is comprised of repeated motifs containing a positively charged amino acid followed by two hydrophobic amino acids and is believed to act as the primary voltage sensor (Yu *et al.* (2005); Bezanilla (2005)).

Voltage gated potassium channels (Kᵥ) are composed of tetramers of the α subunit with each resembling one of the homologous domains of the Caᵥ and Naᵥ channels (Tempel *et al.* (1987), Pongs *et al.* (1988)). A number of other ion channel families contain this structural motif including calcium-activated potassium (K_{Ca}) channels, cyclic nucleotide-gated (CNG) channels, hyperpolarization-activated cyclic nucleotide-modulated (HCN) channels and transient receptor potential (TRP) channels (Yu *et al.* (2005)).

The simplest VGIC structurally is exemplified by the inwardly rectifying potassium ion channels (Kᵢᵣ) that are comprised of four subunits, each having two transmembrane segments termed M1 and M2 that are analogous in structure and function to the S5 and S6 TM segments in the six TM segment Naᵥ, Caᵥ and Kᵥ ion channels (Ho *et al.* (1993); Kubo *et al.* (1993)). The final structural variation consists of the two pore motif potassium channels (K_{2P}) that link together two M1 and M2 TM segments (Goldstein *et al.* (1996); Lesage *et al.* (1996)).

Physiologically, VGICs can be associated with auxiliary subunits that can influence expression of the channel, trafficking to the cell surface plasma membrane and modulation of voltage gating (Isom *et al.* (1994)) although the α is sufficient to generate functional ion conducting channels in heterologous recombinant expression systems. Trafficking of VGICs to the cell surface where they exert their effect is an important regulated event in human cells that has been linked to disease when proper ion channel distribution is interrupted (McCleskey and Gold (1999); Morello *et al.* (2000); Valdivia *et al.* (2002); Manganas *et al.* (2001a); Manganas *et al.* (2001b); Rea *et al.* (2002); Mantegazza *et al.* (2010)). The α pore-forming subunit can contain various signal sequences, such as ER retention signals, that will prevent export of the channel from the ER and subsequent trafficking to the cell surface (Swanwick *et al.* (2010); Vacher and Trimmer (2012)). Auxiliary subunits have been shown to associate with the α subunit of ion channels and directly affect the expression levels and cell surface trafficking of the ion channel by masking α subunit ER retention signals (Zhang *et al.* (2008)).

The varied roles of VGICs in physiological processes, not surprisingly, suggests many therapeutic indications, collectively known as channelopathies, that are associated with aberrant or absent VGIC function. These include conditions affecting neuronal excitability *(e.g.,* pain, epilepsy), heart *(e.g.,* arrhythmias) and muscle contraction *(e.g.,* paralysis). Additionally, VGICs are being investigated as potential targets for therapeutic intervention in autoimmune disease (Judge *et al.* (2006); Sun and Li (2013)) and cancer where multiple studies have shown VGIC involvement in proliferation, migration and survival of a variety of cancer cells (Fiske *et al.* (2006)).

Despite significant effort in developing ion channel therapeutics, targeting indications with an estimated market of $24 billion or more, there have been few VGIC modulating drugs approved by the FDA over the last decade (Hogg *et al.* (2006)). Historically, the majority of ion channel drug development has focused on identifying and developing small molecule modulators. Small molecule drug discovery is typically carried out by screening compound libraries using analytical outputs that show direct modulation of the target ion channel or, alternatively, a binding event that is further characterized for ion channel effects.

The accepted standard for ion channel functional characterization is electrophysiology that allows real time kinetic and pharmacological analysis of the effects of drug molecule candidates. While electrophysiology is the most detailed analytical tool available for ion channel functional modulation and is key in making hit-to-lead candidate determinations, it is resource intensive and has suffered historically from low throughput. However, progress has been made with increasing screening throughput with, for example, platforms that utilize robotic multi-patch clamp configurations (Patchliner^{®}, Nanion Technologies, Munich, DE) or population patch clamp technology (Ion Work^{®} Quattro^{™}, Molecular Devices Corp., Sunnyvale, CA, USA) as well as the development of screens with high throughput capabilities including flux assays, atomic absorption spectroscopy, and fluorescence and colorimetric based assays. While random compound library screening continues to be a focus in ion channel drug discovery efforts, attempts at rational drug design based on VGIC three-dimensional structural information has been limited by a lack of solved human VGIC structures. A major factor limiting progress on determining human VGIC structure has been the low levels of recombinant VGIC produced in mammalian cell lines (e.g., HEK-293). This, in turn, represents a bottleneck in generating enough starting material to carry out successful purification of a VGIC that yields sufficient material (typically >10-20 mg) to enter, for example, crystallization trials that will ultimately generate crystals of a quality suitable for X-ray diffraction analysis.

While mammalian VGICs are highly hydrophobic, complex proteins, previous studies have shown that they can be purified from native sources in a way that maintains functionality of the purified protein following reconstitution into proteoliposomes. For example, a number of labs in the early to mid 1980's purified voltage-gated sodium ion channels from rat brains (Hartshorne *et al.* (1983)) and from the electroplax of *Electrophorus electricus* (Miller *et al.* (1983)), and were able to reconstitute functional activity from purified components (Rosenberg *et al.* (1983); Talvenheimo *et al.* (1984); Tamkun *et al.* (1984); Trainer *et al.* (1993)). More recently, a structure for a voltage-gated sodium channel from *Arcobacter butzleri* has been solved to 3.2 Å resolution (Payandeh *et al.* (2012)). Taken together, these results suggest that solving the crystal structure of human VGICs should be feasible if a source of recombinant protein is available for purification of the ion channel. US 2005/202539 A1 provides methods and compositions for expressing eukaryotic membrane proteins, such as ion channel proteins in ciliates. WO 01/14403 A1 relates to the K + channel MinK2 protein, its transfection into *Paramecium* as well as methods for the screening of compounds that are agonistic or antagonistic to K+ activity. Similarly document WO 00/49048 A1 relates to the gene encoding NDAE1, which is a channel ion protein, as well as methods for the screening of compounds that are agonistic or antagonistic to pHi, Na + or anion channel/ transporter activity.

An important consideration of small molecule development, whether it is from rational design or random screening, is the specificity a potential drug has for a target. With respect to VGICs, this has been particularly challenging as many ion channels belong to families with closely associated members having high levels of homology, particularly within pore-forming domains where many ion channel blockers exert their effect, but nonetheless have significantly different physiological roles. For example, the sodium channel isoforms Na(v)1.7, Na(v)1.8 and Na(v)1.9 have been identified as targets in nociceptor neurons whose modulation ameliorates different pain states. However, strict counter screens are required to characterize potential modulators of these channels for effects on other Nav family members, such as Nav1.5, which is primarily found in cardiac muscle and is responsible for the initial upstroke of the action potential in an electrocardiogram (Zimmer and Surber (2008)).

An obvious alternative to small molecule promiscuity is the development of biologics such as monoclonal antibodies (mAbs) that have high levels of specificity. Despite the appeal of this approach and significant efforts in developing ion channel mAb drug candidates, there has been even less success than there has been in identifying bona fide small molecule drug candidates. There are several challenging aspects of developing modulating ion channel mAbs that are likely the cause of the paucity of success in this endeavor. The first challenge is that, despite the large mass (>150 kDa) of many of the Naᵥ, Caᵥ and Kᵥ VGICs, the targeted extracellular loops, where mAbs are most likely to elicit a modulating effect, are comparatively small and not highly immunogenic, thus hampering immunization efforts. The second challenge is the lack of suitable antigen sources for either immunization programs or antibody screening efforts. Attempts at generating ion channel mAbs via immunization have mostly relied on DNA-based antigens, whole cell or membrane fractions of recombinant mammalian cell lines expressing VGICs, or peptide-based antigens that preferably mimic a targeted extracellular loop structure. Each of these approaches has drawbacks that have thus far contributed to the difficulty in generating ion channel mAbs. Firstly, due to toxicity associated with high levels of expression, recombinant mammalian cell lines typically produce low levels of surface localized, functional recombinant VGICs, which significantly diminishes the antigenic load of cell-based immunogens. Furthermore, cell-based immunogens often include many potential antigens derived from the cell itself that are likely to be more abundant and possibly more immunogenic than the recombinant ion channel, and particularly the associated extracellular loops, making it difficult to generate an ion channel antibody titer that is high enough to effectively screen. DNA-based immunogens are likely to produce a protein that is functional without the added non-specific cell associated antigens of cell-based immunogens, however the yield of protein would be expected to be low and the resultant immune response may not be sufficiently robust to generate a mAb titer high enough to identify potential modulators. Peptide-based antigens usually do not suffer from issues of quantity as they can typically be produced via chemical synthesis or robust cell expression systems such as *E. coli.* However, the physiological relevance of peptide-based antigens, even those that are 3-dimensionally accurate representations of surface loop structures, will always be limited as they lack the context of other molecular determinants associated with the ion channel antigen that may be necessary to generate a high titer of neutralizing mAbs.

Similarly, antibody-screening applications require sufficient levels of physiologically relevant ion channel antigen that will allow high enough resolution to identify binders. Current mammalian cell-based sources of ion channel antigen and peptide-based antigens are inadequate to enable such screens for the reasons mentioned above.

Clearly, a source of recombinant, physiologically relevant, VGICs at significantly higher cell surface density than is currently available in mammalian cell lines would further enable VGIC drug discovery programs involving rational drug design, small molecule screening and monoclonal antibody development utilizing both immunization and screening strategies.

### SUMMARY OF THE INVENTION

In one aspect, the invention provides transgenic ciliates comprising a transgene encoding a mammalian voltage-gated ion channel operably joined to regulatory sequences such that the ciliate expresses the voltage-gated ion channel protein.

The resting membrane potential of the ciliate differs from the resting membrane potential of a healthy native cell in which the voltage-gated ion channel protein is expressed by at least 10 mV, 15 mV or 20 mV. In some embodiments, the resting membrane potential of the ciliate is -15 mV to -40 mV, or -25 mV to -35 mV. In certain embodiments, the resting potential is approximately -30 mV. The resting membrane potential of the ciliate is less polarized (*i.e.,* closer to zero) than the healthy native cell in which the voltage-gated ion channel protein is expressed.

In some embodiments, the native cell is a mammalian cell selected from the group consisting of a skeletal muscle cell, cardiac muscle cell, smooth muscle cell, astrocyte, neuron, lymphocyte, kidney cell and ovarian cell. In some embodiments, the native cell is an immortalized mammalian cell selected from the group consisting of a HEK cell, CHO cell or Jurkat cell.

In some embodiments, the transgenic ciliate produces glycoproteins including asparagine-linked glycans that lack sialic acid residues. In some embodiments, the ciliate produces glycoproteins that comprise at least one glycan selected from the group consisting of Man₅-, Man₄- and Man₃-GlcNAc₂.

In some embodiments, the transgenic ciliate a *Tetrahymena* species, and in particular embodiments the species is *T. thermophila.*

In some embodiments, the regulatory sequences comprise a promoter that is activated by a shift to starvation conditions, such as a promoter that regulates the expression of a gene selected from Table 2.

In some embodiments, regulatory sequences comprise an inducible metallothionein promoter, such as a promoter that regulates the expression of a gene selected from Table 3.

In some embodiments, the promoter is endogenous to ciliates, in some embodiments the genus is *Tetrahymena* and, in particular embodiments the species is *T. thermophila.*

In some embodiments, the voltage-gated ion channel comprises a pore-forming α subunit, and in some embodiments the voltage-gated ion channel is substantially free of auxiliary sub-units. In some embodiments, the voltage-gated ion channel is selected from the group consisting of sodium, calcium and potassium voltage gated ion channels, and in some embodiments the voltage-gated ion channel is selected from the voltage-gated ion channels in Table 4.

In some embodiments, (a) adenine and thymine nucleotides comprise at least 55% of the nucleotides of the coding sequence of the transgene encoding the mammalian voltage-gated ion channel; or (b) at least 50% of the codons of the coding sequence of the transgene encoding the mammalian voltage-gated ion channel are chosen from the favored codons for expression in the ciliate.

In another aspect, the invention provides methods for producing a recombinant mammalian voltage-gated ion channel in a transgenic ciliate of the invention (described above), by providing the transgenic ciliate and culturing it under conditions which permit or induce expression of the voltage-gated ion channel.

Disclosed herein but not part of the claimed invention are lipid bilayers comprising a mammalian voltage-gated ion channel, and tetrahymenol (which is naturally present in the cell membranes of ciliates, including *Tetrahymenta spp*.).

In some instances, the lipid bilayer is substantially free of cholesterol, and in some instances the lipid bilayer is substantially free of other mammalian proteins.

In some instances, the lipid bilayer is derived from a non-mammalian cell, in some instances the cell is a ciliate, in certain instances the genus is Tetrahymena and, in particular instances the species is *T. thermophila.*

In some instances, the voltage-gated ion channel comprises a pore-forming a subunit, and in some instances the voltage-gated ion channel is substantially free of auxiliary sub-units. In some instances, the voltage-gated ion channel is selected from the group consisting of sodium, calcium and potassium voltage gated ion channels, and in some instances the voltage-gated ion channel is selected from the voltage-gated ion channels in Table 4. In some instances, the voltage-gated ion channel comprises glycans selected from the group consisting of Man5-, Man4- and Man3-GlcNAc2.

In some instances, the lipid bilayer comprises a cell surface enriched pellicle fraction derived from a transgenic ciliate expressing the mammalian voltage-gated ion channel.

Disclosed herein but not part of the claimed invention is a population of membrane vesicles comprising a lipid bilayer as described above. In some instances, at least 50% of the membrane vesicles are oriented right-side-out and are derived from a transgenic ciliate expressing the mammalian voltage-gated ion channel. In some instances, greater than 60% of the membrane vesicles have an average diameter of between 30 and 200 nm. In some instances, the membrane vesicles were clarified by an extrusion process.

Disclosed herein but not part of the claimed invention are compositions comprising at least 75% by weigh of a mammalian voltage-gated ion channel, wherein the voltage-gated ion channel is a glycoprotein that lacks sialic acid residues. In some instances, the the voltage-gated ion channel comprises at least one glycan selected from the group consisting of Man₅-, Man₄- and Man₃-GlcNAc₂. In some instances, the voltage-gated ion channel comprises a pore-forming α subunit, and in some instances, the voltage-gated ion channel is substantially free of auxiliary sub-units. In some instances, the composition is substantially free of other mammalian proteins.

In some instances, the voltage-gated ion channel is selected from the group consisting of sodium, calcium and potassium voltage gated ion channels, and in some instances the voltage-gated ion channel is selected from the voltage-gated ion channels in Table 4.

In some instances the voltage-gated ion channel is reconstituted into a liposome comprising phospholipids to produce a proteoliposome, and in some instances, the proteoliposomes have been clarified by an extrusion process.

Disclosed herein but not part of the claimed invention are immunogens comprising the transgenic ciliate of the invention, the lipid bilayers, the populations of membrane vesicles, or the compositions as described above and herein.

Disclosed herein but not part of the claimed invention are methods for producing antibodies that modulate the activity of a voltage-gated ion channel using the immunogen of the disclosure.

Disclosed herein but not part of the claimed invention are methods for producing therapeutic antibodies using an immunogen disclosed herein that modulates the activity of the target voltage-gated ion channel.

Disclosed herein but not part of the claimed invention are methods for screening compound libraries for small molecules that bind and modulate the activity of mammalian voltage-gated ion channels using the transgenic ciliates of the invention, as described above and herein.

Disclosed herein but not part of the claimed invention methods for screening compound libraries for small molecules that bind and modulate the activity of mammalian voltage-gated ion channels using the lipid bilayers, the population of membrane vesicles, or the compositions of as described above and herein.

Aspects of the invention will be apparent to one of ordinary skill in the art from the following detailed description, figures, examples and appended claims.

### BRIEF DESCRIPTION OF THE DRAWINGS

The following drawings are illustrative of embodiments of the invention and are not meant to limit the scope of the invention which is defined by the appended claims.

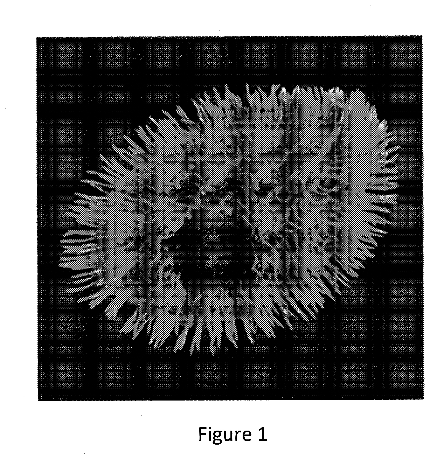

Figure 1. Immunofluorescence light micrograph of *T. thermophila.* The cell is stained with anti-tubulin antibodies to visualize the cilia (hair-like projections at the cell periphery) and DAPI to visualize the nucleus (round body at lower center). The cell dimensions are -20 x 50 microns.
Figure 2. Structural architecture of voltage-gated ion channels. Shown are structural elements of (a) voltage-gated sodium and calcium channels showing four domains each consisting of six transmembrane segments (S1-S6) with a re-entrant loop between S5 and S6; (b) voltage-gated potassium channel showing a single six transmembrane segment (S1-S6) with a re-entrant loop between S5 and S6 that can form homo- or heterotetramers; (c) inwardly-rectifying potassium channel comprising of two transmembrane segments (M1 and M2) joined by a re-entrant loop and; (d) two-pore motif potassium channel consisting of four transmembrane segments (M1-M4) containing re-entrant loops between M1/ M2 and M3/M4. Parallel black lines represent the lipid bilayer and orientation is denoted with an O (outside) and I (inside). In each case amino- and carboxy-terminals are located inside the cell.

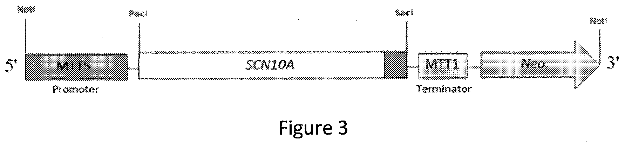

Figure 3. SCN10A *Tetrahymena* expression cassette. Shown from 5' to 3'are the MTT5 promoter, the SCN10A gene modified with a carboxy-dual FLAG/10XHis tag-DYKDDDDKHHHHHHHHHH (Gray Box), the MTT1 terminator and a neomycin resistance cassette (Neoᵣ). The expression cassette is flanked by NotI restriction sites.

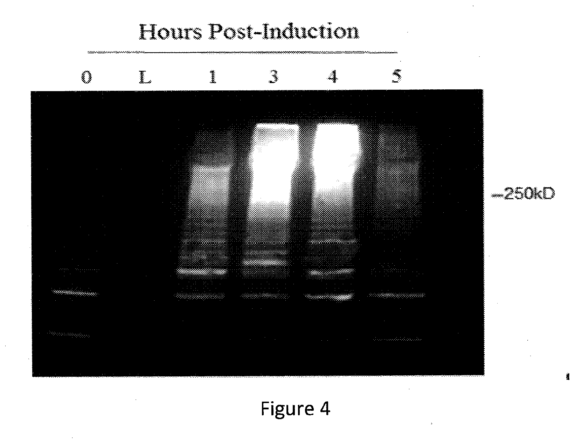

Figure 4. Nav1.8 expression time-course. *Tetrahymena* cells were grown and induced to express Nav1.8 by the addition of CdCl₂ to the growth media. Cells were harvested at 0, 1, 3, 4 and 5 hours post-induction and lysates prepared, standardized for cell number and resolved by SDS-PAGE. Nav1.8 expression was detected by Western blot analysis using an anti-Pan Nav antibody. Peak expression occurs between 3 and 4 hours. L denotes the lane where protein markers were resolved.

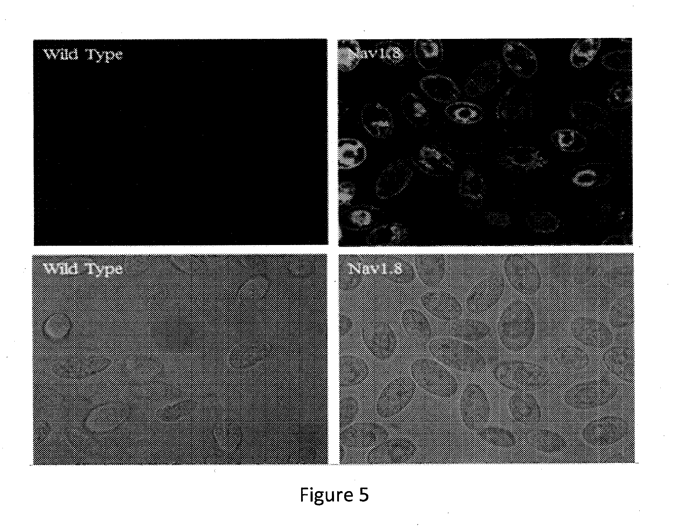

Figure 5. Localization of Nav1.8. *Tetrahymena* cells were grown and induced with CdCl₂. Wild type cells were similarly grown and mock induced. Four hours post-induction cells were harvested, fixed with paraformaldehyde and incubated with anti-FLAG antibody and then a FITC-conjugated mouse IgG antibody. Cells were analyzed by confocal microscopy using an SP5 Leica laser scanning confocal microscope with a 63X oil objective lens (top panels) and by light microscopy (bottom panels). Note that Nav1.8 is detected at both the cell periphery and internally in a perinuclear staining pattern. No staining is observed in wild type cells.

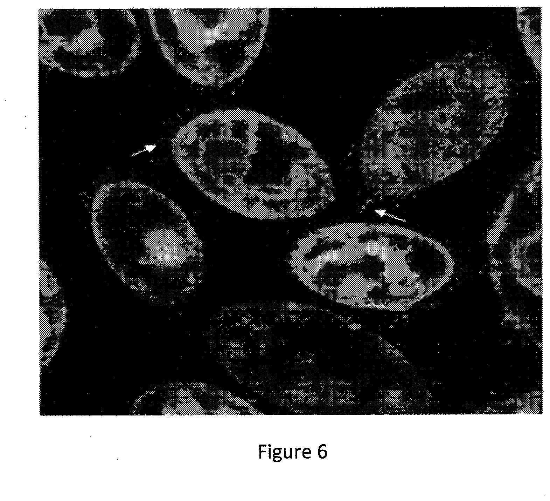

Figure 6. Navl.8 is localized in *Tetrahymena* cilia. Shown is a close up immunofluorescence image of *Tetrahymena* cells expressing Nav1.8. Arrows highlight detection of Navl.8 in cilia.

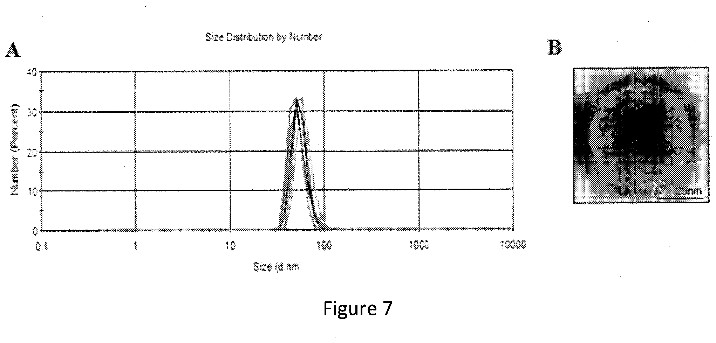

Figure 7. Nav1.8 containing membrane vesicles are highly uniform. A. Dynamic Light Scattering (DLS) analysis of Nav1.8 membrane vesicles. Note that a majority of particles (>90%) fall in the range of 20-150 nm diameter. B. Negative stain electron microscopy image of a Nav1.8 membrane vesicle. Note that the diameter of the membrane vesicle is in general agreement with the DLS data in A.

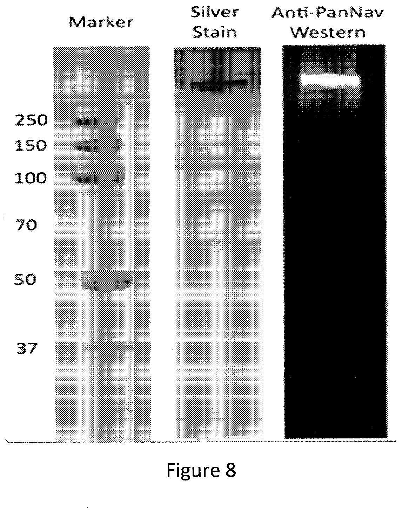

Figure 8. Characterization of purified Nav1.8. Nav1.8 was purified from *Tetrahymena* cell membranes by tandem Nickel affinity and anti-FLAG affinity chromatography as described in the Examples section. Purified Nav1.8 was reconstituted into phosphatidyl-choline containing liposomes to form Nav1.8 proteoliposomes. Proteoliposomes were resolved by SDS-PAGE and protein detected by silver stain (middle panel) and additionally transferred to nitrocellulose and Nav1.8 detected by Western analysis as described above (right panel). Protein size markers are shown in the left panel.

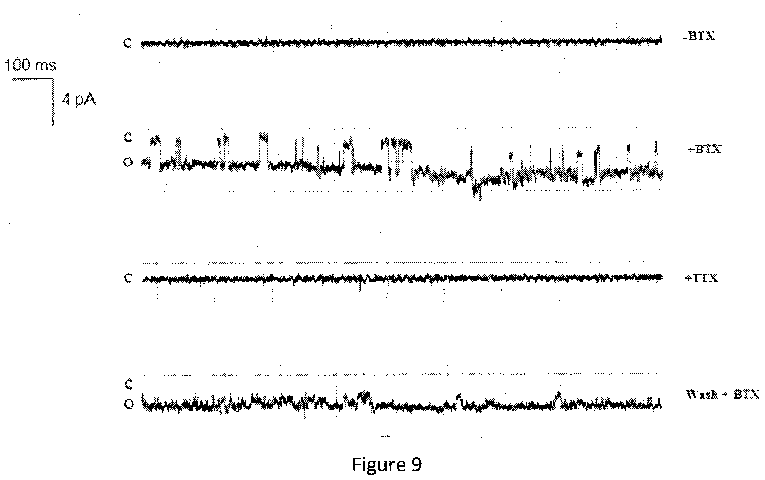

Figure 9. Purified reconstituted Nav1.8 is functional. Navl.8 containing proteoliposomes were fused to artificial bilayers created by Giant Unilammelar Vesicles (GUVs) on a Nanion Port-a-Patch system in a 10x NaCl chemical gradient and 0 mV holding potential. Baseline recordings were established prior to the addition of the neurotoxin activator batrachatoxin (-BTX) where no conductance (*i.e*., activity) was noted. Following the addition of BTX (+BTX) channel activity was detected as exemplified by the appearance of conductances of approximately 2-3 pA. Activity was abolished following the addition of 100 µM Tetrodotoxin (+TTX), a potent sodium channel blocker, and re-established following repeated washing of the system and addition of BTX (Wash+BTX). Note Nav1.8 is a TTX resistant channel but activity is blocked at high (*e.g.,* 100 µM) concentrations. The state of channel activity is highlighted by c (closed) and o (open) symbols. Relative scale of time in milliseconds (ms) and conductance in pico-amps (pA) is shown at left.

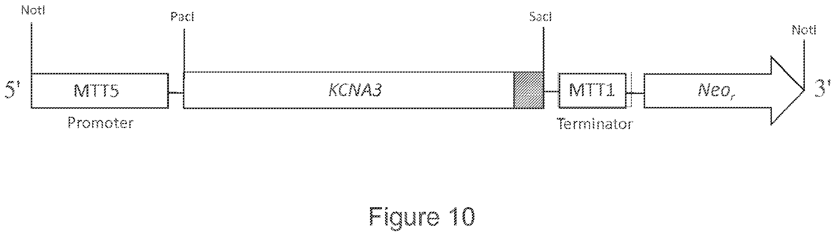

Figure 10. KCNA3 *Tetrahymena* expression cassette. Shown from 5' to 3' are the MTT5 promoter, the KCNA3 gene modified with a carboxy-dual FLAG/10XHis tag-DYKDDDDKHHHHHHHHHH (gray box), the MTT1 terminator and a neomycin resistance cassette (Neoᵣ). The expression cassette is flanked by NotI restriction sites.

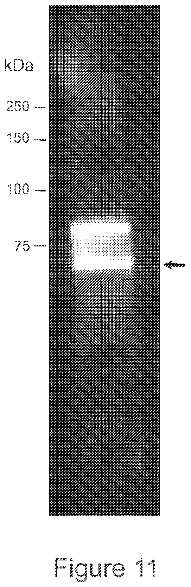

Figure 11. *Tetrahymena* Kvl.3 expression. *Tetrahymena* cells induced to express the KCNA3 gene were harvested and solubilized directly in protein loading buffer. Proteins were resolved by SDS-PAGE and transferred to nitrocellulose membrane. The membrane was probed with anti-Kv1.3 antibody followed by a HRP-conjugated secondary antibody. A band is observed at the approximate predicted mass (~64kDa; highlighted by arrow) as well as a slightly higher molecular weight band (~80kDa). The higher molecular weight band may represent a glycosylated form of Kv1.3 as has been reported in the literature.

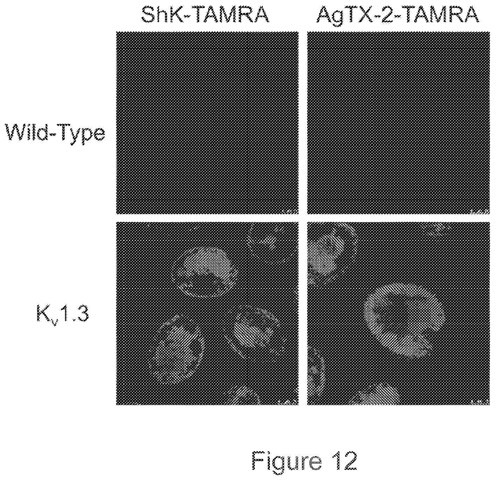

Figure 12. Kv1.3 is located on the *Tetrahymena* cell surface. Wild-type (WT: Top Panels) and Kvl.3 expressing cells (Bottom Panels) were fixed and treated with either ShK-TAMRA (Left Panels) or AgTX-2-TAMRA (Right Panels) and visualized by fluorescence confocal microscopy. No fluorescence is detected in WT cells. However, Kv1.3 is detected on the cell surface of *Tetrahymena* cells when labeled with either ShK- or AgTX-2-TAMRA. (Contrast and brightness of images enhanced for grayscale printing.)

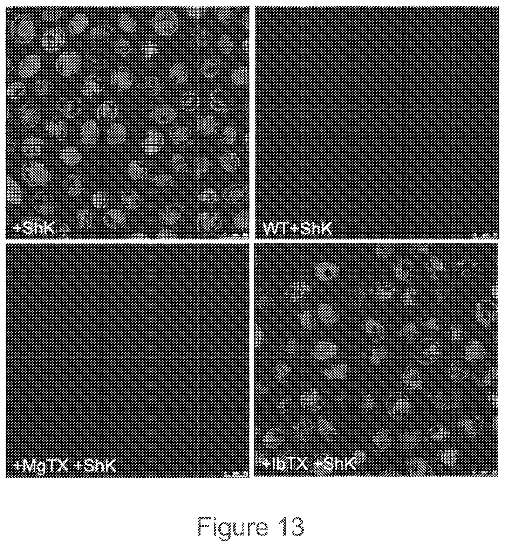

Figure 13. ShK-TAMRA binding to *Tetrahymena* expressed Kv1.3 is specific. Wild-type (WT) or Kv1.3 expressing cells were fixed and treated with 10nM ShK-TAMRA (Top Panels). Kv1.3 expressing cells were additionally incubated in the presence of saturating amounts (100nM) of either the Kv1.3 specific MgTX or non-specific IbTX (Bottom Panels). Note that cell surface labeling is clearly visible in Kv1.3 cells treated with ShK-TAMRA alone but not when co-incubated with MgTX. Co-incubation with a non-specific toxin (IbTX) does not interfere with ShK-TAMRA binding. (Contrast and brightness of images enhanced for grayscale printing.)

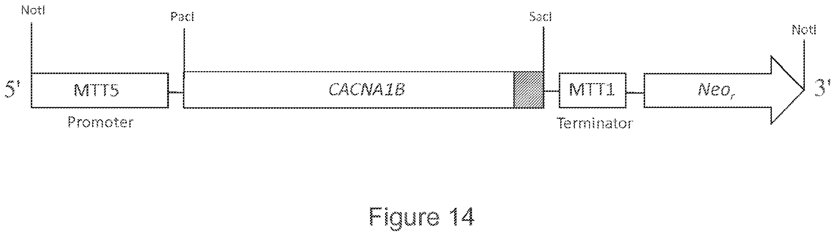

Figure 14. CACNA1B *Tetrahymena* expression cassette. Shown from 5' to 3' are the MTT5 promoter, the CACNA1B gene modified with a carboxy-dual FLAG/10XHis tag-DYKDDDDKHHHHHHHHHH (gray box), the MTT1 terminator and a neomycin resistance cassette (Neoᵣ). The expression cassette is flanked by NotI restriction sites.

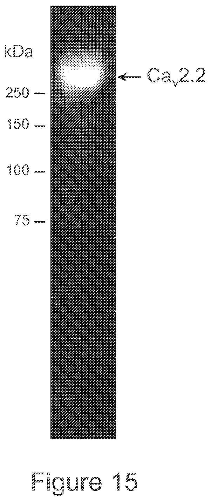

Figure 15. *Tetrahymena* Cav2.2 expression. *Tetrahymena* cells induced to express the CACNA 1B gene were harvested and solubilized directly in protein loading buffer. Proteins were resolved by SDS-PAGE and transferred to nitrocellulose membrane. The membrane was probed with anti-Cav2.2 antibody followed by a HRP-conjugated secondary antibody. A single band is observed at the approximate predicted mass (~270kDa) of Cav2.2 (highlighted by arrow).

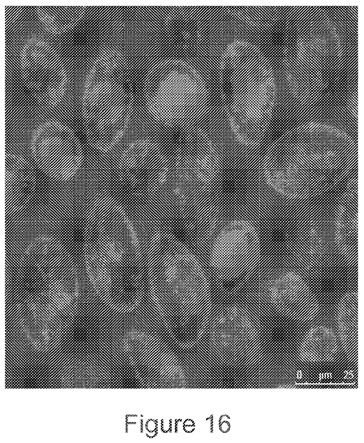

Figure 16. Cav2.2 is expressed on the *Tetrahymena* cell surface. *Tetrahymena* cells expressing Cav2.2 were fixed and probed with anti-Cav2.2 antibody followed by a FITC-conjugated secondary antibody and then examined by confocal microscopy. Like Nav1.8 and Kv1.3 cells show clear cell surface as well as internal perinuclear staining patterns. (Contrast and brightness of image enhanced for grayscale printing.)

### DETAILED DESCRIPTION

The patent, scientific and technical literature referred to herein establish knowledge that was available to those skilled in the art at the time of filing.

In the case of any inconsistencies, the present disclosure will prevail.

### Definitions.

All scientific and technical terms used herein, unless otherwise defined below, are intended to have the same meaning as commonly understood by one of ordinary skill in the art. References to techniques employed herein are intended to refer to the techniques as commonly understood in the art, including variations on those techniques or substitutions of equivalent or later-developed techniques which would be apparent to one of skill in the art. In addition, in order to more clearly and concisely describe the subject matter which is the invention, the following definitions are provided for certain terms which are used in the specification and appended claims.

As used herein, the term "ciliates" means eukaryotes belonging to the kingdom *Chromalveolata,* the superphylum *Alveolata,* and the phylum *Ciliophora.* Ciliates are complex protozoa characterized by the presence of cilia on their cell surfaces and dimorphic nuclei consisting of a macronucleus and one or more micronuclei. Exemplary genera of ciliates are the *Tetrahymena spp.* and the *Paramecium spp.*

As used herein, the term *"Tetrahymena spp."* refers to ciliated protozoa in the family of *Tetrahymenidae.* Exemplary *Tetrahymena spp.* include, but are not limited to, *T. thermophila* and *T. pyriformis.*

As used herein, the term "Paramecium spp." refers to ciliated protozoa in the family of *Parameciidae*. Exemplary Paramecium spp. include, but are not limited to, *P. tetraurelia, P. aurelia, P. caudatum* and *P. bursaria.*

As used herein, the term "heterologous" means, with respect to two or more nucleic acids, proteins or other biomolecules, that the biomolecules do not occur within the same species in nature. For example, a genetic construct may include a coding sequence which is operably joined to one or more regulatory sequences, and/or to one or more other coding sequences, and these sequences are considered heterologous to each other if they are not found in the same species in nature. Similarly, a protein may include a first polypeptide which is joined to a second polypeptide, and these polypeptides are considered heterologous to each other if they are not found in the same species in nature. Thus, a human voltage-gated ion channel coding sequence and a ciliate promoter sequence are heterologous to each other. Similarly, a human voltage-gated ion channel is heterologous with respect to a ciliate plasma membrane.

As used herein, the term "endogenous" means, with respect to nucleic acids, proteins or other biomolecules, that the molecules occur in nature in a reference cell or species. For example, a genetic construct may include a coding sequence which is operably joined to one or more regulatory sequences, and the regulatory sequences are considered endogenous to each other if they are operably joined to the coding sequence in nature, and/or they are considered endogenous to a species if they are found in the same species in nature. Similarly, a protein that occurs naturally in a specified cell type or species is considered endogenous to that cell or species. Thus, a human voltage-gated ion channel coding sequence and a ciliate promoter sequence are heterologous to each other, but the promoter is endogenous to the ciliate.

As used herein, the term "homolog" means a protein which is evolutionarily-related to and shares substantial structural and functional similarity with a reference protein in a different species (*e.g.,* human and mouse homologs of a voltage-gated ion channel).

As used herein, the term "sequence identity" means the percentage of identical nucleotide or amino acid residues at corresponding positions in two or more sequences when the sequences are aligned to maximize sequence matching, *i.e*., taking into account gaps and insertions. The comparison of sequences and determination of percent identity between two sequences can be accomplished using a mathematical algorithm. Techniques for determining sequence identity are well known to one skilled in the art. For purposes of this definition, percentage sequence identity can be determined using the sequence comparison algorithm FASTA, version 3.0t78, using default parameters. See also Pearson and Lipman (1988), Proc Natl. Acad. Sci. USA 85(8):2444-8.

As used herein, the term "promoter" means a nucleotide sequence which is capable of binding an RNA polymerase and initiating transcription of a downstream or 3' coding sequence.

As used herein, the term "transform" means to introduce into a cell a heterologous nucleic acid or nucleic acid analog which replicates within that cell, that encodes a polypeptide sequence which is expressed in that cell (with or without integration into the genome of the cell), and/or that is integrated into the genome of that cell so as to affect the expression of a genetic locus within the genome. The term "transform" is used to embrace all of the various methods of introducing such nucleic acids or nucleic acid analogs, including, but not limited to the methods referred to in the art as transformation, transfection, transduction, or gene transfer, and including techniques such as microinjection, DEAE-dextran-mediated endocytosis, calcium phosphate coprecipitation, electroporation, liposome-mediated transfection, biolistic bombardment, viral-mediated transfection, and the like.

As used herein, the term "transgenic" refers to a cell or organism which has been transformed with a heterologous nucleic acid, causing expression under appropriate conditions of a heterologous protein encoded by the heterologous nucleic acid.

As used herein, the term "vector" means any genetic construct, such as a plasmid, phage, transposon, cosmid, chromosome, virus, virion, etc., which is capable of transferring nucleic acids between cells. Vectors may be capable of one or more of replication, expression, insertion or integration, but need not possess each of these capabilities. Thus, the term includes cloning, expression, homologous recombination, and knock-out vectors. An "expression" vector for a particular heterologous nucleic acid is a vector which can under appropriate conditions cause the encoded protein to be expressed or biosynthesized within the transformed host cell.

As used herein, the term "regulatory sequence" means a DNA transcriptional and translational control sequence such as a promoter, enhancer, polyadenylation signal, terminator, splice signal, and the like, that provide for the expression of a coding sequence as a protein in a host cell.

As used herein, the term "pellicle" means the thin layer supporting the cell membrane in ciliates, protecting them and allowing them to retain their shape, especially during locomotion. The pellicle is formed primarily from closely packed vesicles called alveoli. The term "pellicle preparation" means a preparation including ciliate pellicles and at least some of the associated membrane structures (*e.g.*, alveolar sacs, cilia).

As used herein in connection with membrane vesicles or proteoliposomes, the term "right-side out" means that the terminus of a transmembrane protein which is normally extracellular in native cells expressing the protein is on the outside of a vesicle, liposome or bilayer, and the terminus which is naturally intracellular in native cells expressing the protein is on the inside of the vesicle or liposome.

As used herein, the term "starvation conditions" means conditions of cell culture for ciliates such the cells remain in the G1 phase of the cell cycle and do not divide. Such conditions can be caused by culturing cells in non-nutrient media (*i.e*., substantially deficient in nutrients generally) or media lacking one or more specific nutrients required for vegetative growth by ciliates. Note that starvation conditions may promote mating reactivity but not subsequent vegetative growth.

As used herein, the terms "increase" and "decrease" mean, respectively, to cause an increase or decrease of at least 5%, as determined by a method and sample size that achieves statistically significance (*i.e.*, *p* < 0.1).

As used herein, the term "statistically significant" means having a probability of less than 10% under the relevant null hypothesis(*i.e., p* < 0.1).

As used herein, the term "clarifying" refers to a process of filtering a heterogeneous population of membrane vesicles and possibly contaminants and debris in order to substantially remove the contaminants and debris and reduce the size heterogeneity of the vesicles by removing larger vesicles. The resulting liquid suspension is more transparent or "clarified."

As used herein, the recitation of a numerical range for a variable is intended to convey that the invention may be practiced with the variable equal to any of the values within that range. Thus, for a variable that is inherently discrete, the variable can be equal to any integer value within the numerical range, including the end-points of the range. Similarly, for a variable that is inherently continuous, the variable can be equal to any real value within the numerical range, including the end-points of the range. As an example, and without limitation, a variable which is described as having values between 0 and 2 can take the values 0, 1 or 2 if the variable is inherently discrete, and can take the values 0.0, 0.1, 0.01, 0.001, ... , 0.9, 0.99, 0.999, or any other real values ≥ 0 and ≤ 2, if the variable is inherently continuous.

As used herein, the use of singular forms of words, and the use of the singular articles "a," "an" and "the," are intended to include and not exclude the use of a plurality of the referenced term unless the context clearly dictates otherwise.

As used herein, unless specifically indicated otherwise, the word "or" is used in the inclusive sense of "and/or" and not the exclusive sense of "either/or."

### Production of Mammalian VIGCs in Ciliates: General Considerations.

The present invention is dependent, in part, upon the discovery that ciliates can be used to express mammalian voltage-gated ion channels at levels which are significantly higher than in other standard expression systems without substantial toxicity to the host cells, and with a high percentage of correctly-folded proteins. This high level of expression leads to a higher density of the VIGCs embedded in the plasma membrane of the ciliates, and allows for the production of membrane fractions and membrane vesicles having higher densities of VIGCs, and higher densities of correctly-folded VGICs, than in other standard expression and purification systems.

Significantly, the invention provides vectors, transformed cells and methods for the production of recombinant mammalian VGICs at sufficiently high levels and of sufficient quality to enable drug discovery programs for both small molecules and large molecule (*i.e*., biologics). Furthermore, the invention provides compositions of mammalian VGICs in membrane preparations and in purified form derived from the transformed ciliates.

### Exemplary Ciliate Species.

The ciliates which are used for expression of mammalian VIGCs can be any ciliate species within the kingdom *Chromalveolata,* the superphylum *Alveolata,* and the phylum *Ciliophora.* Exemplary genera of ciliates are the *Tetrahymena spp.* and the *Paramecium spp.* Exemplary *Tetrahymena spp.* include, but are not limited to, *T. thermophila* and *T. pyriformis.* Exemplary *Paramecium spp.* include, but are not limited to, *P. tetraurelia, P. aurelia, P. caudatum* and *P. bursaria.*

Ciliates are complex protozoa characterized by the presence of cilia on their cell surfaces and dimorphic nuclei consisting of a macronucleus and one or more micronuclei. The ciliates have elaborate membrane systems and devote a large part of their metabolism towards membrane protein production. Figure 1 provides a glimpse of the hyper-extended surface of a representative ciliate, *T. thermophila.* As with the microvilli of the intestinal brush border, the hundreds of cilia produced by these cells dramatically increase their surface area. Just beneath the plasma membrane is a second system of alveolar sacs (exclusive to ciliates, apicomplexans and dinoflagellates), which surround the cell. This alveolar membrane system is presumed to function as a Ca⁺⁺ storage compartment (Plattner *et al.* (2012)). In addition, some ciliates produce thousands of membrane delimited dense core granules capable of regulated discharge (Turkewitz *et al.* (2000); Turkewitz (2004)) that add substantially to the total membrane compartment of the cell.

In addition to their complex membrane systems, most ciliates can be grown easily and rapidly in the laboratory. For example, *Tetrahymena* can be cultured at temperatures ranging from 15°C to 41°C in a variety of nutritional media, including several peptone-based media; nutritionally complete, totally defined synthetic medium; skim milk-based medium; and, bacterized medium. Cells can be grown in microliter volumes, but with a generation time of 1.5-3 hrs at 30°C, large volume cultures are also quickly established and simply maintained. Although long-term, exclusively vegetative growth generally results in germline senescence (*e.g*., cultures with decreasing fertility after 12 to 18 months) vegetative clones of interest can be easily frozen in liquid nitrogen for long-term storage (Simon (1982)). For example, although *Tetrahymena* is usually grown in shaker flasks on the bench top, perfused bioreactors will support cell concentrations as high as 2.2 X 10⁷ cells/ml (equivalent to a dry weight of 54 g/L, (Hellenbroich *et al.* (1999)), and industrial media and fermentation processes for growing *Tetrahymena* in 1200 liter suspensions have been described (Hellenbroich *et al.* (1999); deConinck *et al.* (2000)).

Because of their complex biology, ease of growth, and unique genetic structure, *Tetrahymena spp.* have been in wide use as model systems since the 1950s (Nanney (1980)). The genetics, development, cell physiology, biochemistry, and ultrastructure of *Tetrahymena spp.* have been intensively investigated (Frankel (2000)) and have yielded numerous insights into a variety of fundamental biological processes, including those with direct relevance to human health. Indeed, the discovery of dynein as the first microtubule-based motor (Gibbons and Rowe (1965)); telomeres and telomerase (Blackburn *et al.* (1989)); RNA self-splicing (Kruger *et al.* (1982)); the role of histone acetylation in transcription (Brownell *et al.* (1996)); and, *in vivo* functions for histone H1 (Shen and Gorovsky (1996)), histone phosphorylation (Dou and Gorovsky (2000)), and tubulin post-translational modification (Xia *et al.* (2000)) were all made in *Tetrahymena.* In recognition of its importance as a model system, the National Institute of General Medical Sciences (NIGMS) and the National Science Foundation (NSF) jointly funded the sequencing of the *Tetrahymena* macronuclear genome. That effort was completed in late 2003 (to 9x coverage) and is publicly accessible through the *Tetrahymena* Genome Database (TGD) maintained at Stanford University.

For production of recombinant VGICs, specifically, *Tetrahymena* has a number of attributes that may allow it to by-pass the limiting factors associated with low expression yields in mammalian expression hosts. For example, in mammalian expression hosts there appears to be an upper limit to how many functional recombinant channels a mammalian cell can localize to its cell surface before those ion channels create a toxic metabolic environment, presumably through the action of functioning channels. No such toxicity is observed in *Tetrahymena,* thereby allowing the accumulation of many more cell surface recombinant channels. In the case of recombinant VGICs, the reason for this may be partly explained by differences in the resting membrane potential of native human cells and *Tetrahymena* expression host cells. For example, in human neurons the resting membrane potential is approximately -70 mV, whereas in *Tetrahymena* the potential is less polarized and is typically -30 mV, and usually -15 mV to -40 mV. Therefore, for VGICs where activation is linked to changes in membrane voltage potential, it is possible that in a human cell line with a more native resting membrane potential, recombinant channels are 'primed' for activation where they are able to exert a toxic effect. In *Tetrahymena,* cell surface localized ion channels would likely be inactive, though still correctly folded, and therefore not exert any metabolic effect.

As noted above the trafficking of VGICs to the cell surface where they exert their effect is an important event in human cells that is regulated by a number of different mechanisms including manipulation of various retention signals by auxiliary subunits. However, ciliates such as *Tetrahymena* does not encode any obvious orthologs of auxiliary sub units and co-expression of the human beta sub-units is not required for cell surface localization of, for example, Nav1.8 (See Figures 5 and 6). A possible reason for this is that the regulatory retention sequences recognized in human cells may not be as efficiently utilized in *Tetrahymena* thus negating the need for by-passing mechanisms provided by the beta-sub units in human cells.

For specific VGIC families, *Tetrahymena* may be an ideal expression host due to minimal background. For example, although *Tetrahymena* encodes a large number of cation ion channels, none are annotated voltage-gated sodium channels. Importantly, there is also a lack of biochemical evidence that *Tetrahymena* harbors sodium VGICs. Therefore the ability to express specific human sodium VGICs in *Tetrahymena* enables the distinct advantage of characterizing and utilizing those channels in the absence of competing sodium channel 'noise'.

Many human VGICs are glycoproteins that are modified with asparagine-linked glycans on extracellular loops. Unlike other expression host cells, such as yeast, that can modify proteins with large, hypermannosylated N-linked glycans, *Tetrahymena* display minimal heterogeneity amongst glycoforms of native glycoproteins that are restricted to Man5-, Man4- and Man3-GlcNAc2 glycans in a roughly 2:2:6 ratio (Taniguchi *et al* (1985); Weide *et al.* (2006)). Moreover, recombinant heterologous glycoproteins produced in *Tetrahymena* can exhibit uniform glycosylation displaying the Man3-GlcNAc2 that represents the tri-mannosyl core common to all eukaryotic N-linked glycans. Therefore, *Tetrahymena* derived VGICs represent a significantly more appealing protein source for structural analysis by methods such as X-ray crystallography. This in turn provides a powerful enabling technology for rational drug design.

### Methods of Expression of Mammalian VGICs in Ciliates.

In one aspect, the invention provides methods for the production of mammalian VGICs in ciliates, such as *Tetrahymena spp.* which can include all or some of the following steps:
1. Optional codon-optimization of the VGIC gene sequence for expression in the chosen species of ciliate
2. Construction of VGIC expression cassette(s) including the VGIC coding sequence for transformation of the chosen species of ciliate
3. Cloning the expression cassette(s) into a ribosomal DNA vector, which is preferably designed to be in high-copy number in the macronucleus and genetically stable
4. Transformation of ciliate host cells
5. Culturing the transformed ciliates and allowing (or inducing, if necessary) expression of the VGIC
6. Producing membrane preparations enriched in the VGIC
7. Optionally purifying the VGIC from whole cell and/or cell surface membrane preparations

### Each of these steps in discussed in more detail below.

### 1. Optimization of the VGIC gene sequence

Ciliates such as *Tetrahymena* have a slightly different codon usage than that of mammalian cells, such as human cells. Additionally, the *Tetrahymena* genome is AT rich compared to human or other mammalian genes. Although not necessary, heterologous genes can be optimized for expression in the host ciliate. For example, for human VGIC α subunits, which can be greater than 6Kb in length, optimization of the gene sequence is recommended. to help maintain the fidelity of the gene sequence during cloning manipulations carried out in bacteria. Indeed, cloned VGIC α sub-unit genes often include unwanted mutations (*e.g*., frameshift mutations) that would render the protein inactive upon expression or result in the production of truncated inactive protein fragments. These mutations presumably arise during cloning manipulations carried out in bacteria because either the gene itself or some expression by-product causes a selective pressure for those genes that have random inactivating mutations. Ciliate (*e.g*., *Tetrahymena spp*.) optimized genes that are AT rich (in accordance with a ciliate genome and codon usage table), however, do not result in selection against wild-type sequences during bacterial manipulations as do the same genes that are optimized for expression in, for example, human cells and that are not AT rich. Thus optimizing heterologous VGIC genes with an AT-rich ciliate codon usage table can allow for easier and faster manipulations of the cloned gene product.

### 2. Construction of VGIC expression cassette(s)

A variety of expression cassettes are known in the art which can be used to express heterologous proteins in ciliates (see, for example, Shang *et al.* (2002); Smith *et al.* (2004); Weide *et al.* (2007); WO 2011/107520 by Hartmann and Apelt; U.S. Pat. No. 6,846,481 to Gaertig et al.; U.S. Pat. No. 8,664,374 to Colussi et al.; and U.S. Pat. Pub. No. US 2012/0107343 by Clark et al.). Any of these expression systems, or equivalent systems currently existing or developed in the future, can be used to express a heterologous mammalian VGIC protein in a ciliate. Significantly, it is disclosed herein that relatively higher levels of mammalian VGIC expression can be achieved in ciliate cells with relatively less toxicity than achieved in some other standard expression systems. In addition, it is disclosed that a substantial percentage of the VGICs appear to have correct folding than achieved in some other standard expression systems.

In some embodiments, the expression system takes advantage of the natural amplification of copy number of rDNA genes in the macronucleus of ciliates. The ribosomal DNA locus, which is comprised of the 5.7, 18S, and 26S ribosomal RNA coding sequences along with origin of replication (ori) and non-transcribed spacer regions, is present as a single copy in the transcriptionally silent germline micronucleus of *Tetrahymena.* During macronuclear development, the entire ribosomal DNA locus is excised from the genome and duplicated to form an inverted tandem repeat. Following the addition of telomeres, the ribosomal DNA becomes amplified (relative to bulk DNA) as an autonomously replicating minichromosome reaching -9,000 copies per cell in the fully developed, transcriptionally active macronucleus. Amplification is driven by the ribosomal DNA origin of replication. Because the origin of replication for the ribosomal DNA locus of the C-strain of *Tetrahymena* has a replicative advantage compared with the same origin from B-strains of *T. thermophila,* it is possible to replace the entire ribosomal DNA complement by introducing a vector containing the C-strain ribosomal DNA locus into B-strain cells at the time that macronuclear development takes place (that is, conjugation). By incorporating a foreign gene into such a ribosomal DNA vector, one can amplify the transgene in parallel with the entire ribosomal DNA locus resulting in high level expression at the RNA and protein levels.

Analysis of expression profiles in the *Tetrahymena* Gene Expression Database (TGED) of each of the annotated *Tetrahymena* cation channels shows some consistency in terms of life cycle specific expression (Xiong *et al.* (2011)). A majority of *Tetrahymena* ion channels show relatively higher levels of expression during conjugation, a biological event that is preceded by metabolic starvation, a stage at which some ion channels also show high levels of expression. Therefore, in some embodiments, a *Tetrahymena* promoter derived from a gene that shows higher levels of expression during starvation, is used for expression of recombinant VGICs. Such a "starvation promoter" can act as an inducible promoter that initiates gene expression after the cells are shifted from a nutrient-rich to a nutrient-deprived culture medium. For example, a variety of media can be used to induce starvation-based expression, including but not limited to Dryl's medium (2mM sodium citrate, 1mM NaH₂PO₄, 1mM Na₂HPO₄, 1.5mM CaCl₂), NKC medium (0.2% NaCl, 0.008% KCl, 0.12% CaCl2), Tris buffer solution, HEPES buffer solution, or even water. Another potentially significant advantage of induction by starvation is that the starvation media is essentially inert and can be completely devoid of any ion channel substrate (*e.g.*, Na⁺, K⁺, ligands, etc.), allowing for a very 'clean' background in which to produce the ion channel devoid of activity and potentially toxic side effects.

Another option for expression cassettes is to place the VGIC under the control of a metallothionein promoter that induces expression following addition of an inducer to the medium during vegetative growth.

The options of inducing VGIC expression under either starvation or vegetative conditions allows for a customized approach to optimized expression conditions on a target to target basis.

### 3. Cloning expression cassettes into a high-copy genetically stable rDNA vector

Expression cassettes placing the control of a recombinant mammalian VGIC under the control of either a starvation or metallothionein promoter are cloned *en masse* into pTRAS, a ribosomal DNA vector (rDNA) described in WO 2010/108183. Besides allowing for very high levels of transgene to be maintained in a *Tetrahymena* cell (up to 18,000 copies per cell), pTRAS also displays higher levels of genetic stability, compared to other rDNA based vectors, when used in conjunction with the selection drug paromomycin.

### 4. Transformation of ciliates

Methods for transforming ciliates have been previously described and consist of, among other methods, microinjection (Tondravi and Yao (1986)), electroporation (Gaertig and Gorovsky (1992)) and particle bombardment (Cassidy-Hanley *et al.* (1997)).

In some embodiments, tungsten particles coated with VGIC rDNA expression vectors are introduced into the ciliate via particle bombardment (Cassidy-Hanley *et al.* (1997)).

### 5. Culturing transformed ciliates and inducing expression of VGIC

The use of ciliates as model organisms for many years has generated multiple descriptions of their growth and maintenance in culture under both non-controlled (for a review see Cassidy-Hanley (2012)) and controlled fermentation conditions (Hellenbroich *et al.* (1999)).

In some embodiments, *Tetrahymena* cells can be grown in a variety of media sources from complex peptone-based media to chemically defined media. For example, *Tetrahymena* is routinely grown in a complex peptone-based media at 30°C.

### 6. Preparations of ciliate membrane preparations enriched in VGIC

Following induction of VGIC gene expression, ciliate cells are harvested and processed in several different ways to produce membrane preparations with different constituents, biophysical characteristics and levels of VGIC enrichment. Importantly, preparations are enriched in surface membrane components that harbor cell surface localized recombinant VGIC.

Pellicle (Cell Ghost) Membrane Preparation: Ciliate pellicle fractions are enriched in ciliate cell surface membranes such as the plasma membrane, alveolar sac membranes and may also be associated with dense core granule mucocysts that are attached just beneath the plasma membrane. Pellicle preparations are generated following gentle physical disruption of the cell in a way that maintains cell shape but allows for the removal of cytosolic components and internal membrane structure. Pellicles are recovered following density-gradient fractionation as has been described previously (Nozawa and Thompson (1971))

Membrane Vesicles: Membrane vesicles are more highly processed membrane preparations than pellicles. Whole cells are disrupted by pressure to produce vesicles derived from surface and internal vesicles in either a right-side-out or inside-out orientation. Following vesicle enrichment by differential centrifugation, a two-phase (e.g., PEG-Dextran) partitioning system can be employed to isolate membrane vesicles derived from the plasma membrane in a right-side-out orientation. This method is adapted from one originally developed for plant based material (Yoshida *et al.* (1983)) although other methods exist to isolate membrane vesicles with a preferred sidedness using, for example, affinity chromatography (Rice and Lindsey (1997)). For example, membrane vesicles derived from *Tetrahymena* cells expressing VGICs routinely contain a 5-10 fold enrichment of VGICs compared to pellicle preparations. Furthermore, size analysis of ciliate membrane vesicles by dynamic light scattering and negative stain electron microscopy reveals vesicles that are highly homogenous with greater than 60% of the membrane vesicles having an average diameter of between 30 and 200 nm. In some embodiments, greater than 70%, greater than 80%, or greater than 90% of particles have an average diameter in the range of 30 and 200 nm. In some embodiments, greater than 70%, greater than 80%, or greater than 90% of particles have an average diameter in the range of 20-150 nm (see Figure 7).

### 7. Purification of VGICs from whole cell and cell surface ciliate membrane preparations

Recombinant VGICs can be purified from both enriched membrane fractions (such as the pellicle fraction) or from whole cells. Purification involves extracting the VGICs from the ciliate membrane with a non-ionic detergent (*e.g*., TX100, DDM, etc.) and purification with any standard affinity based technique (*e.g*., dual affinity chromatography steps such as nickel and anti-FLAG antibody resins). During purification steps, detergent and phospholipid additives are typically maintained in buffers to ensure VGIC stability. At the completion of purification, the VGICs can be present in a mixed micelle composed of protein, detergent and phospholipid. The purified protein optionally can be processed further by removal of the detergent and reconstitution into phospholipid-containing liposomes to create proteoliposomes.

### EXAMPLES

The following experimental examples relate to exemplary embodiments of the invention including: (a) the expression in *Tetrahymena thermophila* of a recombinant form of the gene SCN10A, which encodes the α sub-unit of the human sodium voltage-gated ion channel protein Nav1.8, the generation of membrane products enriched in the α sub-unit of recombinant Nav1.8 protein, and the purification of the α sub-unit of recombinant Nav1.8, (b) the expression in *Tetrahymena thermophila* of the gene KCNA3 which encodes the human potassium voltage-gated ion channel subunit Kv1.3, and (c) the expression in *Tetrahymena thermophila* of the gene CACNA1B that encodes the α sub-unit of the human calcium voltage-gated ion channel protein Cav2.2.

These examples illustrate some preferred modes of practicing the present invention, but are not intended to limit the scope of the claimed invention. Alternative materials and methods may be utilized to obtain similar results, and the invention can be used to express many other mammalian voltage-gated ion channels by substituting the appropriate gene for SCN10A, KCNA3 or CACNA1B.

SCN10A Gene Construct Design, Synthesis and Cloning: A synthetic gene was designed wherein the Nav1.8 α subunit coding region of the human SCN10A gene containing a C-terminal dual FLAG (DYKDDDDK) and 10X His epitope tag was optimized for expression in *Tetrahymena thermophila.* The GC content of the native human gene (NM_006514.2) is 50.69% whereas the GC content of the *Tetrahymena* optimized gene is 40.25%. The gene was chemically synthesized and cloned into a somatic expression vector, pTIEV5, via PacI and SacI restriction sites engineered into the 5' and 3' ends of the synthetic gene, respectively. Transcription of the transgene was under the control of an optimized cadmium-inducible promoter from the metallothionein-5 (MTT5) gene of *Tetrahymena thermophila.* The expression construct comprising MTT5 promoter, transgene, MTT1 terminator and a neomycin resistance cassette (Figure 3) were transferred, en masse, as a NotI fragment into a high-copy rDNA vector, pTRAS1 and introduced into conjugating *Tetrahymena thermophila* strains by biolistic transformation.

Generation of expression strains: B2086 and CU428 *T. thermophila* strains were grown in modified NEFF medium (0.25% proteose peptone, 0.25% yeast extract, 0.55% glucose, 33 mM FeCl₃) at 30°C. One hundred ml of each logarithmically growing culture was centrifuged at 1,100 x g for 2 minutes in oil centrifuge tubes, washed in 10 mM Tris pH 7.4 and resuspended in fresh 10 mM Tris pH 7.4 (starvation medium) at a concentration of 200,000 - 250,000 cells/ml. Cells were incubated for 9-18 hours at 30°C. After starvation, B2086 and CU428 cell cultures were counted and cell concentration was readjusted to 200,000 cells/ml. To induce conjugation, 100 ml of each strain were mixed together in a 4 L flask. Four transformations were performed between 9.5 and 10.5 hours post-mixing using a Biolistic PDS-1000/He Particle Delivery System (BIO-RAD). For each transformation, 20 µl of M17 (BioRad) tungsten bead suspension in sterile water (60 mg/ml) were coated with 4 µg of DNA construct. Fifty ml of conjugating cells were concentrated to ~1 ml by centrifugation at 1,100 x g in oil centrifuge tubes for 2 minutes. Cells were spread on a round 90 mm hardened paper filter (Whatman, Cat. # 1450-090) pre-wet with 1.5 ml 10 mM Tris pH 7.4 inside a Petri dish. After the bombardment, the filter with the cells was transferred into a 500 ml flask containing 50 ml NEFF medium. The flasks were incubated on a slow shaker for ~20 hours at 30°C. At 30 hours post-mixing, 25 ml NEFF medium containing 300 mg/ml paromomycin was added to the 50 ml of cell culture (final paromomycin concentration, 100 mg/ml). Cells were aliquoted into 96 well microplates (150 ml per well). After 3-4 days, the microplates were examined and 5 ml from each of the wells containing paromomycin-resistant cells were transferred into 150 ml NEFF medium containing 100 mg/ml paromomycin on a master 96 well microplate. To identify Nav1.8 expressing clones, 70 drug-resistant clones were grown to ~5 x 10⁵ cells/ml and induced for 6 hr with 1 µg/ml of CdCl₂. Proteins were resolved by SDS-PAGE and transferred to nitrocellulose membranes before Western blotting. Blots were probed with either an anti-FLAG antibody that recognizes the linear FLAG epitope engineered into the Nav1.8 C-terminus or an anti-PanNav antibody that recognizes a common epitope amongst members of the Nav family of ion channels. Following incubation in primary antibody, blots were probed with secondary goat anti-mouse IgG coupled to HRP for visualization. Once expressing transformants had been identified a time-course was carried out to determine peak expression post-induction. Figure 4 shows that Nav1.8 expression peaks at approximately 3-4 hours post-induction.

Nav1.8 cell localization determination: To determine the cellular localization of recombinant Nav1.8, *Tetrahymena* cells were grown and induced with 2µg/ml CdCl₂ and fixed in 4% paraformaldehyde. Cells were incubated in blocking solution (3% BSA in PBS) for 1-2 hours and then incubated with anti-FLAG antibody (1/100 in blocking solution) for 1 hour at RT, washed and incubated in blocking solution overnight. Cells were subsequently incubated with FITC-conjugated anti-mouse IgG antibody (1/200 in blocking solution) for 1 hour, washed and analyzed by confocal microscopy using an SP5 Leica laser scanning confocal microscope with a 63X oil objective lens. Wild-type control cells were grown, mock induced, fixed, labeled and imaged in the same way as cells expressing Nav1.8.

Nav1.8 is detected at the plasma membrane as well as inside the cell in what appears to be largely perinuclear ER staining indicating that recombinant Nav1.8 trafficks to the *Tetrahymena* cell surface following translation (Figure 5). No immunofluorescence signal was detected in control wild-type cells. Closer inspection of confocal images indicates that Nav1.8 is present in membranes of cilia (Figure 6).

Preparation of surface membrane pellicle fraction enriched in Nav1.8: A 10 ml culture of Nav1.8 expressing cells was started by transferring 0.5 - 1 ml from a stock tube into NEFF medium containing 100 ng/ml paromomycin (pm). Cultures were grown at 30°C with 80-100 rpm shaking, 16 - 36 hrs. One to five ml of the 10 ml culture was transferred into a 1 L flask containing 100 ml NEFF 100 ng/ml pm (1x pm from a 1000x pm stock) and cells incubated for 16-24 hours under the same conditions. Five ml of the 100 ml culture was used to inoculate a 1-2L NEFF culture and cells incubated for 20-24 hours under the same conditions. When the cell density reached 0.8 - 1.0 x 10⁶ cells/ml, gene expression was induced by the addition of CdCl₂ to a final concentration of 1 mg/ml. Cells were further incubated at 30°C with shaking for 6-7 hours. Prior to cell harvesting, 10 sucrose gradients in 50 ml tubes were prepared and kept on ice: bottom layer - 15 ml 1.7 M sucrose in Phosphate Buffer, middle layer - 15 ml 1 M sucrose in Phosphate Buffer, top layer - 10 ml 0.34 M sucrose in Phosphate Buffer. At 4 hours post-induction, the cells were counted and 1 ml samples removed for protein expression analysis. At 4.5 hours post-induction, the cells were harvested by centrifugation: 1,600 x g for 5 minutes at room temperature in an RC-5B centrifuge with an F9-4x1000y rotor. The supernatant was decanted and the pellet(s) were resuspended in 500-700 ml 10 mM Tris-HCl pH 7.4 (pre-warmed to 30°C). Then cells were then spun again under the same conditions. The cell pellet was quickly chilled on ice (less than a minute) and 90 ml ice-cold Phosphate Buffer (0.2 M sodium phosphate pH 7.2, 0.1 M NaCl, 3 mM EDTA) was added. Cells were resuspended by swirling, while the bottle with cells was kept on ice. All the following procedures were performed in the cold room or on ice using ice-cold buffers. The cell density was determined and adjusted to 9 - 10 x 10⁶ cells/ml with Phosphate Buffer. Cells were subjected to loose douncing with an "A" grade douncer. Four to six strokes were initially used and the cells examined by microscopy. If more than 10% of the cells still retain cilia a further 2 strokes were used. The cells were harvested by centrifugation (5 minutes at 1,000 x g at 4°C). The cell pellet containing deciliated cells were resuspended in 50 ml Phosphate Buffer supplemented with one tablet of EDTA-free Protease Inhibitors and 200 ml Turbo DNase, in the cold room, just by inverting the tube or using a 10 ml pipette. The cells were then disrupted using a tight douncer ("B" grade) with between 40-100 strokes. The cell lysis/pellicle release was monitored using a microscope. The homogenate was distributed evenly to 10 x 50 ml tubes containing sucrose gradients (5-7 ml homogenate per gradient, the homogenate was carefully deposited to the top of the gradient, using P1000 pipetteman or disposable plastic pipettes). Gradients were spun in an RT1 centrifuge at 2,700 x g, 15 minutes at 4°C. The 0.34 M and 1 M sucrose layers were discarded while the whitish band of pellicles at the interface between 1.0 M and 1.7 M sucrose (should be about 80 ml from 10 gradients) was collected. The pellicle containing solution was diluted with an equal volume of phosphate buffer and distributed to three or four 50 ml tubes and spun at 4,080 x g, for 20 minutes at 4°C. The supernatant was discarded and the pellets were resuspended in 30-35 ml Resuspension Buffer (20 mM Tris-HCl pH 7.4, 0.1 M NaCl), combined all in one 50 ml tube, and the volume adjusted to 40 ml. The number of pellicles in the 40 ml suspension was determined using a hemacytometer. The pellicles were harvested by centrifugation (4,080 x g, 20 minutes at 4°C). The supernatant was discarded and the pellet frozen in liquid nitrogen and stored, at -80°C.

Preparation of membrane vesicles enriched in Nav1.8: A 1L culture of cells were grown and induced to express Nav1.8. Cells were harvested by centrifugation and resuspended in 45 or 180ml of Mucus Secretion Buffer (40mM Hepes, 2mM CaCl₂ pH 7.4). Mucocyst secretion was induced by the addition of 5 ml dibucaine per/L of culture initially grown. Samples were centrifuged at 2700g for 10 minutes. The cell pellet was retained and resuspended in 100ml potassium phosphate buffer (200mM pH 7.2) containing protease inhibitors . The suspension was microfluidized one time at 1 KPsi and centrifuged at 1000 x g for 10 min. The supernatant was collected and further centrifuged at 10,000 x g for 20 minutes. The resulting supernatant was further centrifuged at 100,000 x g for 1 hour. The 100K x g pellet was resuspended in 5 ml potassium phosphate buffer and stored at -80°C until further processing. The frozen pellet was thawed and dounce homogenized until no particles were visible. Two grams of resuspended pellet were loaded onto a previously prepared PEG/Dextran two-phase solution (6.6% w/v dextran in 0.2 M potassium phosphate, pH 7.2 and 6.6 PEG w/v dextran in 0.2 M potassium phosphate, pH 7.2). The sample was vortexed for several seconds and allowed to equilibrate at 4°C for 20 minutes and centrifuged in a clinical centrifuge for 20 min at 4°C. The top layer was harvested and diluted with an equal volume of 1 x PBS and centrifuged at 100K x g for 1 h at 4°C. The pellet was resuspended in approximately 100 µl of 1 x PBS and total protein measured by BCA assay. Total protein concentration was adjusted to 1 mg/ ml with 1 x PBS. Biophysical characterization of membrane vesicles was carried out by dynamic light scattering (DLS) using a Malvern Zetasizer Nano ZS90 and negative stain electron microscopy (Figure 7).

Purification of Nav1.8: Nav1.8 can be purified from either whole cells or from enriched membrane fractions. Following extraction of Nav1.8, purification via affinity chromatography proceeds in a similar manner regardless of the source material.

Extraction of Nav1.8 from Pellicles: Thaw frozen pellicle, prepared as described above, on ice in 10 ml resuspension buffer (1XPBS, 100 mM choline chloride, 10% glycerol, 20 mM imidazole, pH7.4, EDTA-free Complete protease inhibitor tables) per pellicle preparation derived from a 1 L culture. Homogenize thawed pellicle in a dounce homogenizer (tight handle). Add an equal volume of extraction buffer (resuspension buffer additionally containing 2% TX100 and 0.5% L-a-phosphatidyl choline from egg yolk Type XVI-E) and incubate on a rocking platform at 4°C for 1 hour. Spin samples at 100,000 x g for 1 hour at 4°C and filter supernatant samples through a 0.45 micron filter. Proceed to Nickel affinity purification.

Extraction of Nav1.8 from Whole Cells: Two liters of Nav1.8 culture was induced with 2µg/ml CdCl₂ for 4hr and harvested by centrifugation. The resulting cell pellet was resuspended in 100ml 1X PBS supplied with protease inhibitors at a concentration 1 tablet per 10ml of cell suspension and Turbo DNase 15µl/20ml of PBS. The resulting cell suspension was distributed equally between 4 x 50ml conical tubes containing 10ml of glass beads, washed and chilled on ice prior to sample addition. Cell suspensions were homogenized by vortexing for 10-20 sec pulses keeping samples on ice between pulses. Cell lysis was checked by light microscopy. When there were no visible cell ghosts the homogenates were left on ice for glass beads to settle, following which the homogenate was transferred to fresh 50ml conical tubes. Cell lysates were spun at 16-20K x g for 30min. The resulting supernatant was discarded and the pellet was resuspended in 100ml PBS supplied with protease inhibitors and DNase and spun again. Extraction of Nav1.8 then proceeded as described above for pellicle preparations.

Affinity Purification of Nav1.8: A bed volume, approximately 10% of the extract volume, of nickel sepharose beads twice washed in equilibration buffer (1 x PBS, 100 mM choline chloride, 10% glycerol, 20 mM imidazole, 1% TX100, 0.25% phosphatidyl choline, pH7.4) was added to the extract and incubated overnight at 4°C on a rocking platform. The sepharose beads were collected by centrifugation and washed three times in 7-8 volumes of wash buffer (1 × PBS, 100 mM choline chloride, 10% glycerol, 0.1% TX100, 0.025% phosphatidyl choline, EDTA-free protease inhibitors, pH 7.4). Bound protein was eluted in 4 column volumes of elution buffer (1 × PBS, 100 mM choline chloride, 10% glycerol, 0.025% TX100, 0.00625% phosphatidyl choline, 250 mM imidazole, EDTA-free protease inhibitors, pH 7.4). The eluted sample was dialyzed overnight at 4°C in >50 volumes dialysis buffer (1 x PBS, 100 mM choline chloride, 10% glycerol, 0.025% TX100, 0.00625% phosphatidyl choline, EDTA-free protease inhibitors, pH 7.4). A bed volume of anti-FLAG M2 affinity gel, approximately 10% of the dialyzed sample, was washed with 2 column volumes low pH buffer (100 mM glycine, 100 mM choline chloride, 150 mM NaCl, 10% glycerol, 0.025% TX100, 0.00625% phosphatidyl choline, pH 3.5) and then twice with 10 column volumes of dialysis buffer. The FLAG affinity resin was added to the dialyzed sample and incubated for 2 hours at 4°C on a rocking platform. The resin was collected by centrifugation and washed three times with 10 column volumes of dialysis buffer. Bound protein was eluted in 2 column volume elutions of elution buffer (50 mM NaPO₄, 150 mM NaCl, 0.025% TX100, 0.00625% phosphatidyl choline, 0.15 mg/ml 3XFLAG peptide, pH 7.4). Elution step was repeated 3 times. Elution fractions were pooled and dialyzed overnight in 100 volumes elution buffer lacking FLAG peptide (50 mM NaPO₄, 150 mM NaCl, 0.025% TX100, 0.00625% phosphatidyl choline, pH 7.4) at 4°C. The dialyzed sample was concentrated approximately 5 fold in a spin column with a 100 kDa MWCO filter. The sample was stored at 4°C until further processing.

Reconstitution of purified Nav1.8 in Proteoliposome: Liposomes were prepared in the following manner (all steps were carried out in glass vessels): 40 mg of phosphatidyl choline dissolved in chloroform was dried down in a glass tube. The film of dried phosphatidyl choline was resuspended in 1 ml NaPO₄ buffer (50 mM NaPO₄, 150 mM NaCl, pH 7.4) to produce a 40 mg/ml solution. The solution went through 3 freeze/thaw cycles using liquid nitrogen followed by a 2 minute period in a sonication bath. The entire process was repeated 3 times. Liposomes were stored on ice until further use.

Detergent from affinity purified Nav1.8 was removed by the addition of 0.2 g/ml SM2 biobeads, pre-hydrated in water for 10 minutes. The sample was incubated on ice for 2-3 hours with occasional gentle swirling. The sample was added to pre-prepared liposomes at a ratio of 0.33 ml liposomes: 1 ml protein sample to give a final phosphatidyl choline concentration of 10 mg/ml. The proteoliposome sample was freeze/thawed 3 times in liquid nitrogen followed by a 2 minute sonication bath. The freeze/thaw was repeated once and the entire sample was frozen and stored at -80°C.

Characterization of purified and reconstituted Nav1.8: Following purification and reconstitution into proteoliposomes, Nav1.8 was characterized by a combination of Western blot analysis (as described above) to ensure recovery of the protein, silver stained SDS-PAGE to estimate purity (Figure 8) and electrophysiology to confirm functionality of the purified protein (Figure 9).

KCNA3 Gene Construct Design. Synthesis and Cloning: A synthetic gene was designed wherein the Kv1.3 subunit coding region of the human KCNA3 gene containing a C-terminal dual FLAG (DYKDDDDK) and 10X His epitope tag was optimized for expression in *Tetrahymena thermophila.* The gene was chemically synthesized and cloned into a somatic expression vector, pTIEV5, via PacI and SacI restriction sites engineered into the 5' and 3' ends of the synthetic gene, respectively. Transcription of the transgene was under the control of an optimized cadmium-inducible promoter from the metallothionein-5 (MTT5) gene of *Tetrahymena thermophila.* The expression construct comprising MTT5 promoter, transgene, MTT1 terminator and a neomycin resistance cassette (Figure 10) were transferred together as a NotI fragment into a high-copy rDNA vector, pTRAS1 and introduced into conjugating *Tetrahymena thermophila* strains by biolistic transformation as described above for SCN10A.

To identify Kv1.3 expressing clones, drug-resistant clones were grown to ~5 x 10⁵ cells/ml and induced for 6 hr with 1 µg/ml of CdCl₂. Proteins were resolved by SDS-PAGE and transferred to nitrocellulose membranes before Western blotting. Blots were probed with either an anti-FLAG antibody that recognizes the linear FLAG epitope engineered into the Kv1.3 C-terminus or a specific anti-Kv1.3 antibody. Following incubation in primary antibody, blots were probed with secondary goat anti-mouse IgG coupled to HRP for visualization. Figure 11 shows a representative anti-Kv1.3Western blot analysis image of Kv1.3 expression in *Tetrahymena* cells.

Kv1.3 cell localization determination: To determine the cellular localization of recombinant Kv1.3, *Tetrahymena* cells were grown and induced with 2µg/ml CdCl₂ and fixed in 4% paraformaldehyde. Cells were incubated in the presence of 10nM fluorescently-tagged Kv1.3 specific toxins Agitoxin-2-TAMRA (AgTX-2-TAMRA) or ShK-TAMRA for 30 minutes at RT. Cells were washed and analyzed by confocal microscopy using an SP5 Leica laser scanning confocal microscope with a 63X oil objective lens. Wild-type control cells were grown, mock-induced, fixed, labeled and imaged in the same way as cells expressing Kv1.3. To determine if the labeling of Kv1.3 expressing cells was specific, cells were also co-incubated with 10nM ShK-TAMRA in the presence of saturating amounts (100nM) of either non-labeled Kv1.3 specific Margatoxin (MgTX) or non-specific calcium activated potassium channel blocker Iberiotoxin (IbTX). Figure 12 shows that both AgTX-2-TAMRA and ShK-TAMRA bound to Kvl.3 expressing cells and that Kv1.3 was localized on the *Tetrahymena* cell surface. No labeling was observed with WT cells. Figure 13 shows that ShK-TAMRA binding was specific and competitive with saturating amounts of the Kv1.3 specific MgTX but not the non-specific IbTX.

CACNA1B Gene Construct Design, Synthesis and Cloning: A synthetic gene was designed wherein the Cav2.2 α subunit coding region of the human CACNA1B gene containing a C-terminal dual FLAG (DYKDDDDK) and 10X His epitope tag was optimized for expression in *Tetrahymena thermophila.* The gene was chemically synthesized and cloned into a somatic expression vector, pTIEV5, via PacI and SacI restriction sites engineered into the 5' and 3' ends of the synthetic gene, respectively. Transcription of the transgene was under the control of an optimized cadmium-inducible promoter from the metallothionein-5 (MTT5) gene of *Tetrahymena thermophila.* The expression construct comprising MTT5 promoter, transgene, MTT1 terminator and a neomycin resistance cassette (Figure 14) were transferred together as a NotI fragment into a high-copy rDNA vector, pTRAS1, and introduced into conjugating *Tetrahymena thermophila* strains by biolistic transformation as described above for SCN10A.

To identify Cav2.2 expressing clones, drug-resistant clones were grown to ~5 x 10⁵ cells/ml and induced for 6 hr with 1 µg/ml of CdCl₂. Proteins were resolved by SDS-PAGE and transferred to nitrocellulose membranes before Western blotting. Blots were probed with an anti-Cav2.2 antibody followed by a secondary goat anti-mouse IgG coupled to HRP for visualization. Figure 15 shows a representative anti-Cav2.2 Western blot analysis image of Cav2.2 expression in *Tetrahymena* cells.

Cav2.2 cell localization determination: To determine the cellular localization of recombinant Cav2.2, *Tetrahymena* cells were grown and induced with 2µg/ml CdCl₂ and fixed in 4% paraformaldehyde. Cells were incubated in blocking solution (3% BSA in PBS) for 1-2 hours and then incubated with anti-Cav2.2 antibody (1/100 in blocking solution) for 1 hour at RT, washed and incubated in blocking solution overnight. Cells were subsequently incubated with FITC-conjugated secondary antibody (1/200 in blocking solution) for 1 hour, washed and analyzed by confocal microscopy using an SP5 Leica laser scanning confocal microscope with a 63X oil objective lens.

Figure 16 shows that, similar to Nav1.8 and Kv1.3, Cav2.2 is detected at the plasma membrane indicating that recombinant Cav2.2 traffics to the *Tetrahymena* cell surface. Similarly fixed and treated WT cells did not show a fluorescence signal (not shown).

### References

Bezanilla F. (2005) Voltage-gated ion channels. IEEE Trans. Nanobioscience. 4:34-48
Blackburn E.H., Greider C.W., Henderson E., Lee M.S., Shampay J., and Shippen-Lentz D. (1989). Recognition and elongation of telomeres by telomerase. Genome 2, 553-560
Brownell J.E., Zhou J., Ranalli T., Kobayashi R., Edmondson D.G., Roth S.Y., and Allis C.D. (1996). Tetrahymena histone acetyltransferase A: a homolog to yeast Gcn5p linking histone acetylation to gene activation. Cell 6:843-851
Cassidy-Hanley D.M. (2012) Tetrahymena in the laboratory: strain resources, methods for culture, maintenance, and storage. Methods Cell Biol. 109:237-76
Cassidy-Hanley D., Bowen J., Lee J.H., Cole E., VerPlank L.A., Gaertig J. Gorovsky M.A. and Bruns P.J. (1997) Germline and somatic transformation of mating Tetrahymena thermophila by particle bombardment. Genetics 146:135-147
de Coninck J., Bouquelet S., Dumortier V., Duyme F. and I. Verdier-Denantes (2000). Industrial media and fermentation processes for improved growth and protease production by Tetrahymena thermophila Bill. J. Industr. Microbiol. Biotechnol. 24, 285
Dou Y., and Gorovsky M.A. (2000). Phosphorylation of linker histone H1 regulates gene expression in vivo by creating a charge patch. Mol. Cell 2:225-31
Eisen J.A., Coyne R.S., Wu M., Wu D., Thiagarajan M., Wortman J.R., Badger J.H., Ren Q., Amedeo P., Jones K.M. et al. (2006). Macronuclear genome sequence of the ciliate Tetrahymena thermophila, a model eukaryote. PLoS Biol 9, e286
Fiske J., Fomin V.P., Brown M.L., Duncan R.L. and Sikes R.A. (2006) Voltage-sensitive ion channels and cancer. Cancer Metastasis Rev. 25:493-500
Frankel J. (2000). Cell biology of Tetrahymena thermophila. Methods Cell Biol. 27-125
Gaertig J. and Gorovsky M.A. (1992) Efficient mass transformation of Tetrahymena thermophila by electroporation of conjugants. PNAS 89:9196-9200
Gibbons I.R. and Rowe A.J. (1965). Dynein: A protein with adenosine triphosphatase activity. Science 424
Goldstein S.A., Price L.A., Rosenthal D.N. and Pausch M.H. (1996) ORK1, a potassium-selective leak channel with two pore domains cloned from Drosophila melanogaster by expression in Saccharomyces cerevisiae. PNAS. 93:13256-13261
Hartshorne R.P. and Catterall W.A. (1983) The sodium channel from rat brain. J. Biol. Chem. 259:1667-1675
Hellenbroich D., Valley U., Ryll T., Wagner R., Tekkanat N., Kessler W., Rob A., and Deckwer W.-D. (1999). Cultivation of Tetrahymena thermophila in a 1.5 m3 airlift bioreactor. Appl. Microbiol. Biotechnol. 51: 447.
Ho K., Nichols C.G., Lederer W.J., Lytton J., Vassilev P.M., Kanazirska M.V. and Hebert S.C. (1993) Cloning and expression of an inwardly rectifying ATP-regulated potassium channel. Nature. 362:31-38
Hogg D.S., Boden P, Lawton G. and Kozlowski R.Z. (2006) Ion channel drug targets; unlocking the potential. Drug Discovery World
Isom L.L., DeJongh K.S. and Catterall W.A. (1994) Auxiliary subunits of voltage-gated ion channels. Neuron. 12:1183-1194
Judge S.I.V., Lee J.M., Bever Jr C.T. and Hoffman P.M. (2006) Voltage-gated potassium channels in multiple sclerosis: Overview and new implications for treatment of central nervous system inflammation and degeneration. J Rehab. Res. Devel.. 43:111-122
Kruger K., Grabowski P.J., Zaug A.J., Sands J., Gottschling D.E., and Cech T.R. (1982). Self-splicing RNA: autoexcision and autocyclization of the ribosomal RNA intervening sequence of Tetrahymena. Cell 1:147-157
Kubo Y., Baldwin T.J., Jan Y.N. and Jan L.Y. (1993) Primary structure and functional expression of a mouse inward rectifier potassium channel. Nature. 362:127-133
Lampert T.J., Coleman K.D. and Hennessey T.M. (2011) A knockout mutation of a constitutive GPCR in Tetrahymena decreases both G-protein activity and chemoattraction. PLoS One. 6:e28022
Lesage F., Guillemare E., Fink M., Duprat F., Lazdunski M., Romey G. and Barhanin J. (1996) TWIK-1, a ubiquitous human weakly inward rectifying potassium channel with a novel structure. EMBO. 15:1004-1011
Manganas L.N., Akhtar S., Antonucci D.E., Campomanes C.R., Dolly J.O. and Trimmer J.S. (2001a) Episodic ataxia type-1 mutations in the Kv1.1 potassium channel display distinct folding and intracellular trafficking properties. J. Biol. Chem. 276:49427-49434
Manganas L.N., Wang Q., Scannevin R.H., Antonucci D.E., Rhodes K.J and Trimmer J.S. (2001b) Identification of a trafficking determinant localized to the Kv1 potassium channel pore. PNAS. 98:14055-14059
Mantegazza M., Curia G., Biagini G., Ragsdale D.S. Avoli M. (2010) Voltage-gated sodium channels as therapeutic targets in epilepsy and other neurological disorders. Lancet Neurol. 9:413-424
McCleskey E.W. and Gold M.S. (1999) Ion channels of nociception. Annu. Rev. Physiol. 61:835-856
Miller J.A., Agnew W.S. and Levinson S.R. (1983) Principal glycopeptides of the tetrodotoxin/saxitoxin binding protein from Electrophorus electricus: isolation and partial chemical and physical characterization. Biochemistry. 22:462-470
Morello J.P., Petaja-Repo U.E., Bichet D.G, and Bouvier M. (2000) Pharmacological chaperones: a new twist on receptor folding. Trends Pharmacol. Sci. 21:466-469
Nanney D.L. (1980). Experimental Ciliatology: An introduction to genetic and developmental analysis in Ciliates (New York, NY: John Wiley & Sons)
Nozawa Y. and Thompson G.A. Jr. (1971) Studies of membrane formation in Tetrahymena pyriformis II. Isolation and lipid analysis of cell fractions. J Cell Biol. 49:712-721
Payandeh J., Gamal El-Din T.M., Scheuer T., Zheng N. and Catterall W.A. (2012) Crystal structure of a voltage-gated sodium channel in two potentially inactivated states. Nature. 486:135-139
Plattner H., Sehring I.M., Mohamed I.K., Miranda K., De Souza W., Billington R., Genazzani A. and Ladenburer E.M. (2012) Calcium signaling in closely related protozoan groups (Alveolata): non-parasitic ciliates (Paramecium, Tetrahymena) vs. parasitic Apicomplexa (Plasmodium, Toxoplasma). Cell Calcium. 51:351-382
Pongs O., Kecskemethy N., Muller R., Krah-Jentgens I., Baumann A., Kiltz H.H., Canal, I., Llamazares, S. and Ferrus A. (1988) Shaker encodes a family of putative potassium channel proteins in the nervous system of Drosophila. EMBO. 7:1087-1096
Rice J.E. and Lindsay J.G. (1997) Subcellular Fractionation: A Practical Approach. (Oxford University Press: J.M. Graham and D. Rickwood Editors)
Rosenberg R.L., Tomiko S.A. and Agnew W.S. (1984) Reconstitution of neurotoxin-modulated ion transport by the voltage-regulated sodium channel isolated from the electroplax of Electrophorus electricus. PNAS. 81:1239-1243
Simon E.M. (1982) Breeding performance of Tetrahymena thermophila following storage for 5 to 6 years in liquid nitrogen. Cryobiology. 19:607-612
Shang Y., Song X., Bowen J., Corstanje R., Gao Y., Gaertig J. and Gorovsky M.A. (2002) PNAS. 99:3734-3739
Shen X., and Gorovsky M.A. (1996). Linker histone H1 regulates specific gene expression but not global transcription in vivo. Cell 3, 475-83
Sun H. and Min L. (2013) Antibody therapeutics targeting ion channels: are we there yet? Acta Pharmacol. Sinica. 34:199-204
Smith J.J., Coles E.S. and Romero D.P. (2004) Transcriptional control of RAD51 expression in the ciliate Tetrahymena thermophila. Nuc. Acids Res. 32:4313-4321
Swanswick R.S., Pristera A.and Okuse K. (2010) The trafficking on Nav1.8. Neuroscience Letters 486:78-83
Talvenheimo J.A., Tamkun M.M., Messner D.J., Hartshorne R.P., Sharkey R.M. and Catterall W.A. (1984) Structure and functional reconstitution of the sodium channel from rat brain. Biophys. J. 45:37-40
Tamkun M.M., Talvenheimo J.A. and Catterall W.A. (1984) The sodium channel from rat brain. Reconstitution of neurotoxin-activated ion flux and scorpion toxin binding from purified components. J. Biol. Chem. 259:1676-1688
Taniguchi, T. Mizuochi, T., Banno, Y., Nozawa, Y. & A. Kobata (1985). Carbohydrates of lysosomal enzymes secreted by Tetrahymena pyriformis. J. Biol. Chem. 260:13941-13946.
Tempel B.L., Papazian D.M., Schwarz T.L., Jan Y.N. and Jan L.Y. (1987) Sequence of a probable potassium channel component encoded at the Shaker locus of Drosophila. Science. 237:770-775
Tondravi M.M. and Yao M-C. (1986) Transformation of Tetrahymena thermophila by microinjection of ribosomal R A genes. PNAS 83:4369-4373.
Trainer V.L., Moreau E., Guedin D., Baden D.G. and Catterall W.A. (1993) Neurotoxin binding and allosteric modulation at receptor sites 2 and 5 on purified and reconstituted rat brain sodium channels. J. Biol. Chem. 268:17114-17119
Turkewitz A.P., Chilcoat N.D., Haddad A., and Verbsky J.W. (2000). Regulated protein secretion in Tetrahymena thermophila. Meth. Cell Biol. 62:347-362
Turkewitz A.P. (2004). Out with a bang! Tetrahymena as a model system to study secretory granule biogenesis. Traffic 5:63-68
Vacher H. and Trimmer J.S. (2012) Trafficking mechanisms underlying neuronal voltage-gated ion channel localization at the axon initial segment. Epilepsia. 9:21-31
Valdivia C.R., Ackerman M.J., Tester D.J., Wada T., McCormack J., Ye B. and Makielski J.C. (2002) A novel SCN5A arrhythmia mutation, M1766L, with expression defect rescued by mexiletine. Cardiovasc. Res. 55:279-289
Weide T., Bockau U., Rave A., Hermann L. and Hartmann M.W. (2007) A recombinase system facilitates cloning of expression cassettes in the ciliate Tetrahymena thermophila. BMC Microbiol. 7:12
Weide T., Herrmann L., Bockau U., Niebur N., Aldag I., Laroy W., Contreras R., Tiedtke A., and Hartmann M.W. Secretion of functional human enzymes by Tetrahymena thermophila (2006). BMC Biotechnol. 6, 19.
Xia L., Hai B., Gao Y., Burnette D., Thazhath R., Duan J., Bre M.H., Levilliers N., Gorovsky M.A. and Gaertig, J. (2000). Polyglycylation of tubulin is essential and affects cell motility and division in Tetrahymena thermophila. J. Cell Biol. 5:1097-106.
Xiong J., Lu X., Lu Y., Zeng H., Yuan D., Feng L., Chang Y., Bowen J., Gorovsky M., Fu C. and Miao W. (2011) Tetrahymena Gene Expression Database (TGED): a resource of microarray data and co-expression analyses for Tetrahymena. Sci China Life Sci. 54:65-67
Yoshida S., Uemura M., Niki T., Sakai A. and Gusta L.V. (1983) Partition of membrane particles in aqueous two-polymer phase system and its practical use for purification of plasma membranes from plants. Plant Physiol. 72:105-114
Yu F.H. and Catterall W.A. (2004) The VGL-chanome: a protein superfamily specialized for electrical signaling and ionic homeostasis. Sci STKE. 2004. 2004, re15
Yu F.H., Yarov-Yarovoy V., Gutman G.A. and Catterall W.A. (2005) Overview of molecular relationships in the voltage-gated ion channel superfamily. Pharm. Rev. 57:387-395
Zhang Z-N., Li Q., Liu C., Wang H-B., Wang Q. and Bao L. (2008) The voltage-gated Na+ channel Navl.8 contains an ER-retention/retrieval signal antagonized by the b3 subunit. J Cell. Sci. 121:3243-3252
Zimmer T. and Surber R. (2008) SCN5A channelopathies-an update on mutations and mechanisms. Prog. Biophys. Mol. Biol. 98:120-136

| **Table 1** | |
|---|---|
| **Cell Type** | **Resting Membrane Potential (mV)** |
| Skeletal Muscle | -95 |
| Smooth Muscle | -50 |
| Astrocytes | -80 to -90 |
| Neurons | -70 |
| Cardiac Muscle | -85 to -90 |
| Lymphocyte | -40 to -70 |
| E. coli | -105 to -140 |
| Yeast | -120 to -180 |
| Plant (e.g. BY2 Tobacco cells) | -120 to -160 |
| Insect (e.g. Sf9 cells) | -50 to -100 |
| HEK 293 | -55 |
| CHO | -50 to -60 |
| Jurkat | -55 to -60 |

| Table 2 | |
|---|---|
| **Gene** | **Accession #** |
| TTHERM_00013600 | XM_001008339 |
| TTHERM_00013620 | XM_001008341 |
| TTHERM_00013640 | XM_001008343 |
| TTHERM_00024150 | XM_001008501 |
| TTHERM_00024160 | XM_001008502 |
| TTHERM_00024230 | XM_001008509 |
| TTHERM_00671930 | XM_001014970 |
| TTHERM_00735400 | XM_001011589 |
| TTHERM_00753320 | XM_001018331 |
| TTHERM_00754720 | XM_001018368 |
| TTHERM_02255850 | XM_001028613 |
| TTHERM_00155630 | XM_001009689 |
| TTHERM_00158010 | XM_001009729 |
| TTHERM_00805870 | XM_001011986 |
| TTHERM_00816260 | XM_001016133 |
| TTHERM_00185390 | XM_001008810 |
| TTHERM_00833720 | XM_001013679 |
| TTHERM_00862740 | XM_001017043 |
| TTHERM_00864910 | XM_001026731 |
| TTHERM_00864920 | XM_001026732 |
| TTHERM_00879290 | XM_001016081 |
| TTHERM_00879300 | XM_001016082 |
| TTHERM_00881360 | XM_001016090 |
| TTHERM_00881380 | XM_001016092 |
| TTHERM_00881390 | XM_001016093 |
| TTHERM_00881410 | XM_001016095 |
| TTHERM_00881420 | XM_001016096 |
| TTHERM_00881440 | XM_001016098 |
| TTHERM_00881450 | XM_001016099 |
| TTHERM_00891190 | XM_001020311 |
| TTHERM_00193300 | XM_001017083 |
| TTHERM_00193310 | XM_001017084 |
| TTHERM_02465150 | XM_001028377 |
| TTHERM_00940290 | XM_001026889 |
| TTHERM_00227590 | XM_001014290 |
| TTHERM_00227620 | XM_001014293 |
| TTHERM_01055590 | XM_001027525 |
| TTHERM_01060860 | XM_971418 |
| TTHERM_01066970 | XM_971435 |
| TTHERM_00251250 | XM_001019075 |
| TTHERM_00251260 | XM_001019076 |
| TTHERM_00252260 | XM_001019077 |
| TTHERM_00257060 | XM_001019161 |
| TTHERM_00257110 | XM_001019166 |
| TTHERM_00257120 | XM_001019167 |
| TTHERM_01087790 | XM_001017871 |
| TTHERM_01144930 | XM_001026130 |
| TTHERM_01205390 | XM_001029966 |
| TTHERM_01220430 | XM_001025768 |
| TTHERM_01250000 | XM_001021805 |
| TTHERM_00049210 | XM_001014816 |
| TTHERM_01349990 | XM_001012555 |
| TTHERM_01353120 | XM_001017329 |
| TTHERM_01353130 | XM_001017330 |
| TTHERM_00338490 | XM_001017612 |
| TTHERM_00339880 | XM_001017652 |
| TTHERM_00191020 | XM_001016679 |
| TTHERM_00191040 | XM_001016681 |
| TTHERM_00191050 | XM_001016682 |
| TTHERM_00191060 | XM_001016683 |
| TTHERM_00129450 | XM_001016414 |
| TTHERM_00467230 | XM_001025019 |
| TTHERM_00525070 | XM_001028017 |
| TTHERM_00376190 | XM_001015398 |
| TTHERM_01508150 | XM_001015167 |
| TTHERM_00418100 | XM_001007271 |
| TTHERM_00428610 | XM_001013841 |
| TTHERM_00079390 | XM_001015780 |
| TTHERM_00449650 | XM_001013360 |
| TTHERM_00450850 | XM_001013381 |
| TTHERM_00450940 | XM_001013390 |
| TTHERM_01658040 | XM_972188 |
| TTHERM_00463820 | XM_001018879 |
| TTHERM_00094270 | XM_001012881 |
| TTHERM_00532380 | XM_001024361 |
| TTHERM_00107030 | XM_001012347 |
| TTHERM_00577290 | XM_001009419 |
| TTHERM_00113080 | XM_001010703 |
| TTHERM_00117560 | XM_001010755 |
| TTHERM_00637310 | XM_001032392 |
| TTHERM_00637330 | XM_001032394 |
| TTHERM_01999410 | |
| TTHERM_00655440 | XM_001032179 |

| Table 3 | |
|---|---|
| **Gene Name** | **GenBank Accession #** |
| MTT1 | EAS04643; GI: 89306655 |
| MTT2 | EAR91133; GI: 89293145 |
| MTT3 | EAS04644; GI: 586727534 |
| MTT4 | EAR91134; GI: 89293146 |
| MTT5 | EAR99841; GI: 89301853 |

| Table 4 | | |
|---|---|---|
| Ion Channel | Gene Name | Accession # |
| **Voltage Gated Sodium** | | |
| Nav1 | NAV1 | Q8NEY1 |
| Nav1.2 | SCN2A | Q99250 |
| Nav1.3 | SCN3A | Q9NY46 |
| Nav1.4 | SCN4A | P35499 |
| Nav1.5 | SCN5A | Q14524 |
| Nav1.6 | SCN8A | Q9UQD0 |
| Nav1.7 | SCN9A | Q15858 |
| Nav1.8 | SCN10A | Q9Y5Y9 |
| Nav1.9 | SCN11A | Q9U133 |
| Nav2.1/Nav2.2 | SCN7A | NP 002967 |
| Nav3 | NAV3 | Q8IVL0 |
| | | |

| **Voltage Gated Potassium** | | |
|---|---|---|
| Kv1.1 | KCNA1 | Q09470 |
| Kv1.2 | KCNA2 | P16389 |
| Kv1.3 | KCNA3 | P22001 |
| Kv1.4 | KCNA4 | P22459 |
| Kv1.5 | KCNA5 | P22460 |
| Kv1.6 | KCNA6 | P17658 |
| Kv1.7 | KCNA7 | Q96RP8 |
| Kv1.8 | KCA10 | Q16322 |
| Kv2.1 | KCNB1 | Q14721 |
| Kv2.2 | KCNB2 | Q92953 |
| Kv3.1 | KCNC1 | P48547 |
| Kv3.2 | KCNC2 | Q96PR1 |
| Kv3.3 | KCNC3 | Q14003 |
| Kv3.4 | KCNC4 | Q03721 |
| Kv4.1 | KCND1 | Q9NSA2 |
| Kv4.2 | KCND2 | Q9NZV8 |
| Kv4.3 | KCND3 | Q9UK17 |
| Kv5.1 | KCNF1 | Q9H3M0 |
| Kv6.1 | KCNG1 | Q9UIX4 |
| Kv6.2 | KCNG2 | Q9UJ96 |
| Kv6.3 | KCNG3 | Q8TAE7 |
| Kv6.4 | KCNG4 | Q8TDN1 |
| Kv7.1 | KCNQ1 | P51787 |
| Kv7.2 | KCNQ2 | O43526 |
| Kv7.3 | KCNQ3 | O43525 |
| Kv7.4 | KCNQ4 | P56696 |
| Kv7.5 | KCNQ5 | Q9NR82 |
| Kv8.1 | KCNV1 | Q6PIU1 |
| Kv8.2 | KCNV2 | Q8TDN2 |
| Kv9.1 | KCNS1 | Q96KK3 |
| Kv9.2 | KCNS2 | Q9ULS6 |
| Kv9.3 | KCNS3 | Q9BQ31 |
| Kv10.1 | KCNH1 | O95259 |
| Kv10.2 | KCNH5 | Q8NCM2 |
| Kv11.1 | KCNH2 | Q12809 |
| Kv11.2 | KCNH6 | Q9H252 |
| Kv11.3 | KCNH7 | Q9NS40 |
| Kv12.1 | KCNH8 | Q96L42 |
| Kv12.2 | KCNH3 | Q9ULD8 |
| Kv12.3 | KCNH4 | Q9UQ05 |
| KvBeta1 | NP 751892 | NP_751892 |
| KvBeta2 | KCNAB2 | |
| | | |

| **Voltage Gated Calcium** | | |
|---|---|---|
| Cav1.1 | CAC1S | Q13698 |
| Cav1.2 | NP_955630 | NP_955630 |
| Cav1.3 | CAC1D | Q01668 |
| Cav1.4 | CAC1F | O60840 |
| Cav2.1 | CAC1A | O00555 |
| Cav2.2 | CAC1B | Q00975 |
| Cav2.3 | CAC1E | Q15878 |
| Cav3.1 | CAC1G | O43497 |
| Cav3.2 | CAC1H | 095180 |
| Cav3.3 | CAC1I | Q9P0X4 |
| cacnb1 | CACB1 | Q02641 |
| cacnb2 | CACB2 | Q08289 |
| cacnb3 | CACB3 | P54284 |
| cacnb4 | CACB4 | O00305 |
| cacng1 | CCG1 | Q06432 |
| cacng2 | CCG2 | Q9Y698 |
| cacng3 | CCG3 | O60359 |
| cacng4 | CCG4 | Q9UBN1 |
| cacng5 | CCG5 | Q9UF02 |
| cacng6 | CCG6 | Q9BXT2 |
| cacnq7 | CCG7 | P62955 |
| cacng8 | CCG8 | Q8WXS5 |
| cacna2d1 | CA2D1 | P54289 |
| cacna2d2 | CA2D2 | Q9NY47 |
| cacna2d3 | CA2D3 | Q8IZS8 |
| | | |

| **Voltage Gated Chloride** | | |
|---|---|---|
| CICn1 | CLCN1 | P35523 |
| CICn2 | CLCN2 | P51788 |
| CICn3 | CLCN3 | P51790 |
| CICn4 | CLCN4 | P51793 |
| CICn5 | CLCN5 | P51795 |
| CICn6 | CLCN6 | P51797 |
| CICn7 | CLCN7 | P51798 |
| CICK1 - voltage sensitive Ka | CLCKA | P51800 |
| CICK2 - Voltage sensitive Kb | CLCKB | P51801 |

## Claims

1. A transgenic ciliate comprising:
a transgene encoding a mammalian voltage-gated ion channel operably joined to regulatory sequences such that the ciliate expresses the voltage-gated ion channel protein,
wherein the resting membrane potential of the ciliate differs from the resting membrane potential of a healthy native cell in which the voltage-gated ion channel protein is expressed by at least 10 mV, wherein the resting membrane potential of the ciliate is less polarized than the healthy native cell in which the voltage-gated ion channel protein is expressed, and
wherein the transgene is present within a ribosomal DNA (rDNA) vector.

2. The transgenic ciliate of claim 1, wherein the resting membrane potential of the ciliate differs from the resting membrane potential of a healthy native cell in which the voltage-gated ion channel protein is expressed by at least 15 mV; or wherein the resting membrane potential of the ciliate differs from the resting membrane potential of a healthy native cell in which the voltage-gated ion channel protein is expressed by at least 20 mV.

3. The transgenic ciliate of claim 1, wherein the resting membrane potential of the ciliate is -15 mV to -40 mV, or -25 mV to -35 mV.

4. The transgenic ciliate of claim 1, wherein the ribosomal DNA vector is pTRAS.

5. The transgenic ciliate of claim 1, wherein the native cell is:
(a) a mammalian cell selected from the group consisting of a skeletal muscle cell, cardiac muscle cell, smooth muscle cell, astrocyte, neuron, lymphocyte, kidney cell and ovarian cell; or
(b) an immortalized mammalian cell selected from the group consisting of a HEK cell, CHO cell or Jurkat cell.

6. The transgenic ciliate of claim 1, wherein the ciliate produces:
(a) glycoproteins including asparagine-linked glycans that lack sialic acid residues; or
(b) glycoproteins that comprise at least one glycan selected from the group consisting of Man₅-, Man₄- and Man₃-GlcNAc₂.

7. The transgenic ciliate of claim 1, wherein the ciliate is a *Tetrahymena* species, optionally wherein the *Tetrahymena* species is *Tetrahymena thermophila.*

8. The transgenic ciliate of claim 1, wherein the regulatory sequences comprise a promoter that is activated by a shift to starvation conditions, optionally wherein the promoter regulates the expression of a gene selected from TTHERM_00013600, TTHERM_00013620, TTHERM_00013640, TTHERM_00024150, TTHERM_00024160, TTHERM_00024230, TTHERM_00671930, TTHERM_00735400, TTHERM_00753320, TTHERM_00754720, TTHERM_02255850, TTHERM_00155630, TTHERM_00158010, TTHERM_00805870, TTHERM_00816260, TTHERM_00185390, TTHERM_00833720, TTHERM_00862740, TTHERM_00864910, TTHERM_00864920, TTHERM_00879290, TTHERM_00879300, TTHERM_00881360, TTHERM_00881380, TTHERM_00881390, TTHERM_00881410, TTHERM_00881420, TTHERM_00881440, TTHERM_00881450, TTHERM_00891190, TTHERM_00193300, TTHERM_00193310, TTHERM_02465150, TTHERM_00940290, TTHERM_00227590, TTHERM_00227620, TTHERM_01055590, TTHERM_01060860, TTHERM_01066970, TTHERM_00251250, TTHERM_00251260, TTHERM_00252260, TTHERM_00257060, TTHERM_00257110, TTHERM_00257120, TTHERM_01087790, TTHERM_01144930, TTHERM_01205390, TTHERM_01220430, TTHERM_01250000, TTHERM_00049210, TTHERM_01349990, TTHERM 01353120, TTHERM 01353130, TTHERM_00338490, TTHERM_00339880, TTHERM_00191020, TTHERM_00191040, TTHERM_00191050, TTHERM_00191060, TTHERM_00129450, TTHERM_00467230, TTHERM_00525070, TTHERM_00376190, TTHERM_01508150, TTHERM_00418100, TTHERM_00428610, TTHERM_00079390, TTHERM _00449650, TTHERM_00450850, TTHERM_00450940, TTHERM_01658040, TTHERM_00463820, TTHERM_00094270, TTHERM_00532380, TTHERM_00107030, TTHERM_00577290, TTHERM_00113080, TTHERM_00117560, TTHERM_00637310, TTHERM_00637330, TTHERM_01999410, or TTHERM_00655440.

9. The transgenic ciliate of claim 1, wherein the regulatory sequences comprise an inducible metallothionein promoter, optionally wherein the promoter regulates the expression of a gene selected from MTT1, MTT2, MTT3, MTT4, or MTT5.

10. The transgenic ciliate of claim 8 wherein the promoter is:
(a) endogenous to ciliates; or
(b) endogenous to *Tetrahymena thermophila.*

11. The transgenic ciliate of claim 1, wherein the voltage-gated ion channel:
(a) comprises a pore-forming α subunit;
(b) is substantially free of auxiliary sub-units;
(c) is selected from the group consisting of sodium, calcium, and potassium voltage gated ion channels; or
(d) is selected from the voltage-gated ion channels Nav1, Navl.2, Navl.3, Navl.4, Nav1.5, Navl.6, Navl.7, Navl.8, Navl.9, Nav2.1/Nav2.2, Nav3, Kv1.1, Kv1.2, Kv1.3, Kv1.4, Kv1.5, Kv1.6, Kv1.7, Kv1.8, Kv2.1, Kv2.2, Kv3.1, Kv3.2, Kv3.3, Kv3.4, Kv4.1, Kv4.2, Kv4.3, Kv5.1, Kv6.1, Kv6.2, Kv6.3, Kv6.4, Kv7.1, Kv7.2, Kv7.3, Kv7.4, Kv7.5, Kv8.1, Kv8.2, Kv9.1, Kv9.2, Kv9.3, Kv10.1, Kv10.2, Kv11.1, Kv11.2, Kv11.3, Kv12.1, Kv12.2, Kv12.3, KvBetal, KvBeta2, Cav1.1, Cav1.2, Cav1.3, Cav1.4, Cav2.1, Cav2.2, Cav2.3, Cav3.1, Cav3.2, Cav3.3, cacnb1, cacnb2, cacnb3, cacnb4, cacng1, cacng2, cacng3, cacng4, cacng5, cacng6, cacng7, cacng8, cacna2d1, cacna2d2, cacna2d3, ClCn1, ClCn2, ClCn3, ClCn4, ClCn5, ClCn6, ClCn7, ClCK1, or ClCK2.

12. The transgenic ciliate of claim 1, wherein:
a) adenine and thymine nucleotides comprise at least 55% of the nucleotides of the coding sequence of the transgene encoding the mammalian voltage-gated ion channel; or
b) at least 50% of the codons of the coding sequence of the transgene encoding the mammalian voltage-gated ion channel are chosen from the favored codons for expression in the ciliate.

13. A method of producing a recombinant mammalian voltage-gated ion channel in a transgenic ciliate comprising:
providing the transgenic ciliate, the transgenic ciliate comprising a transgene encoding a mammalian voltage-gated ion channel operably joined to regulatory sequences such that the ciliate expresses the voltage-gated ion channel protein,
wherein the resting membrane potential of the ciliate differs from the resting membrane potential of a healthy native cell in which the voltage-gated ion channel protein is expressed by at least 10 mV, wherein the resting membrane potential of the ciliate is less polarized than the healthy native cell in which the voltage-gated ion channel protein is expressed, wherein the transgene is present within a ribosomal DNA (rDNA) vector; and
culturing the transgenic ciliate under conditions which permit or induce expression of the voltage-gated ion channel.

14. The transgenic ciliate of claim 1, wherein the ciliate comprises about 9,000 copies of the rDNA vector.

## Patentansprüche

1. Transgener Ciliat, umfassend:
ein Transgen, das für einen spannungsabhängigen Ionenkanal eines Säugers codiert, der funktionsfähig mit regulatorischen Sequenzen verbunden ist, sodass der Ciliat das spannungsabhängige Ionenkanalprotein exprimiert,
wobei sich das Ruhemembranpotential des Ciliaten von dem Ruhemembranpotential einer gesunden nativen Zelle, in der das spannungsabhängige Ionenkanalprotein exprimiert wird, um mindestens 10 mV unterscheidet, wobei das Ruhemembranpotential des Ciliaten weniger polarisiert ist als das der gesunden nativen Zelle, in der das spannungsabhängige Ionenkanalprotein exprimiert wird, und
wobei das Transgen innerhalb eines ribosomalen DNA-Vektors (rDNA-Vektors) vorhanden ist.

2. Transgener Ciliat nach Anspruch 1, wobei sich das Ruhemembranpotential des Ciliaten von dem Ruhemembranpotential einer gesunden nativen Zelle, in der das spannungsabhängige Ionenkanalprotein exprimiert wird, um mindestens 15 mV unterscheidet; oder wobei sich das Ruhemembranpotential des Ciliaten von dem Ruhemembranpotential einer gesunden nativen Zelle, in der das spannungsabhängige Ionenkanalprotein exprimiert wird, um mindestens 20 mV unterscheidet.

3. Transgener Ciliat nach Anspruch 1, wobei das Ruhemembranpotential des Ciliaten -15 mV bis -40 mV oder -25 mV bis -35 mV beträgt.

4. Transgener Ciliat nach Anspruch 1, wobei der ribosomale DNA-Vektor pTRAS ist.

5. Transgener Ciliat nach Anspruch 1, wobei die native Zelle Folgendes ist:
(a) eine Säugerzelle, ausgewählt aus der Gruppe bestehend aus einer Skelettmuskelzelle, einer Herzmuskelzelle, einer glatten Muskelzelle, einem Astrozyten, einem Neuron, einem Lymphozyten, einer Nierenzelle und einer Ovarialzelle; oder
(b) eine immortalisierte Säugerzelle, ausgewählt aus der Gruppe bestehend aus einer HEK-Zelle, einer CHO-Zelle oder einer Jurkat-Zelle.

6. Transgener Ciliat nach Anspruch 1, wobei der Ciliat Folgendes produziert:
(a) Glycoproteine, die Asparagin-verknüpfte Glycane einschließen, denen Sialinsäurereste fehlen; oder
(b) Glycoproteine, die mindestens ein Glycan umfassen, ausgewählt aus der Gruppe bestehend aus Man₅-, Man₄- und Man₃-GlcNAc₂.

7. Transgener Ciliat nach Anspruch 1, wobei der Ciliat eine *Tetrahymena*-Art ist, wobei die *Tetrahymena*-Art optional *Tetrahymena thermophila* ist.

8. Transgener Ciliat nach Anspruch 1, wobei die regulatorischen Sequenzen einen Promotor umfassen, der durch eine Umstellung auf Hungerbedingungen aktiviert wird, wobei der Promotor optional die Expression eines Gens reguliert, ausgewählt aus
TTHERM_00013600, TTHERM_00013620, TTHERM_00013640, TTHERM_00024150, TTHERM_00024160, TTHERM_00024230, TTHERM_00671930, TTHERM_00735400, TTHERM_00753320, TTHERM_00754720, TTHERM_02255850, TTHERM_00155630, TTHERM_00158010, TTHERM_00805870, TTHERM_00816260, TTHERM_00185390, TTHERM_00833720, TTHERM_00862740, TTHERM_00864910, TTHERM_00864920, TTHERM_00879290, TTHERM_00879300, TTHERM_00881360, TTHERM_00881380, TTHERM_00881390, TTHERM_00881410, TTHERM_00881420, TTHERM_00881440, TTHERM_00881450, TTHERM_00891190, TTHERM_00193300, TTHERM_00193310, TTHERM_02465150, TTHERM_00940290, TTHERM_00227590, TTHERM_00227620, TTHERM_01055590, TTHERM_01060860, TTHERM_01066970, TTHERM _00251250, TTHERM_00251260, TTHERM_00252260, TTHERM_00257060, TTHERM_00257110, TTHERM_00257120, TTHERM_01087790,
TTHERM_01144930, TTHERM_01205390, TTHERM_01220430, TTHERM_01250000, TTHERM_00049210, TTHERM_01349990, TTHERM_01353120, TTHERM_01353130, TTHERM_00338490, TTHERM_00339880, TTHERM_00191020, TTHERM_00191040, TTHERM_00191050, TTHERM_00191060, TTHERM_00129450, TTHERM_00467230. TTHERM_00525070, TTHERM_00376190, TTHERM_01508150, TTHERM_00418100, TTHERM_00428610, TTHERM_00079390, TTHERM_00449650, TTHERM_00450850, TTHERM_00450940, TTHERM_01658040, TTHERM_00463820, TTHERM_00094270, TTHERM_00532380, TTHERM_00107030, TTHERM_00577290, TTHERM_00113080, TTHERM_00117560, TTHERM_00637310, TTHERM_00637330, TTHERM_01999410 oder TTHERM_00655440.

9. Transgener Ciliat nach Anspruch 1, wobei die regulatorischen Sequenzen einen induzierbaren Metallothionein-Promotor umfassen, wobei der Promotor optional die Expression eines Gens reguliert, ausgewählt aus MTT1, MTT2, MTT3, MTT4 oder MTT5.

10. Transgener Ciliat nach Anspruch 8, wobei der Promotor Folgendes ist:
(a) endogen zu Ciliaten; oder
(b) endogen zu *Tetrahymena thermophila.*

11. Transgener Ciliat nach Anspruch 1, wobei der spannungsabhängige Ionenkanal:
(a) eine porenbildende α-Untereinheit umfasst;
(b) im Wesentlichen frei von Hilfsuntereinheiten ist;
(c) ausgewählt ist aus der Gruppe bestehend aus spannungsabhängigen Natrium-, Calcium- und Kaliumionenkanälen; oder
(d) ausgewählt ist aus den spannungsabhängigen Ionenkanälen Nav1, Nav1.2, Nav1.3, Nav1.4, Nav1.5,
Nav1.6, Nav1.7, Nav1.8, Nav1.9, Nav2.1/Nav2.2, Nav3, Kv1.1, Kv1.2, Kv1.3, Kv1.4, Kv1.5, Kv1.6, Kv1.7, Kv1.8, Kv2.1, Kv2.2, Kv3.1, Kv3.2, Kv3.3, Kv3.4, Kv4.1, Kv4.2, Kv4.3, Kv5.1, Kv6.1, Kv6.2, Kv6.3, Kv6.4, Kv7.1, Kv7.2, Kv7.3, Kv7.4, Kv7.5, Kv8.1, Kv8.2, Kv9.1, Kv9.2, Kv9.3, Kv10.1, Kv10.2, Kv11.1, Kv11.2, Kv11.3,Kv12.1, Kv12.2, Kv12.3, KvBeta1, KvBeta2, Cav1.1, Cav1.2, Cav1.3, Cav1.4, Cav2.1, Cav2.2, Cav2.3, Cav3.1, Cav3.2, Cav3.3, cacnb1, cacnb2, cacnb3, cacnb4, cacng1, cacng2, cacng3, cacng4, cacng5, cacng6, cacng7, cacng8, cacna2d1, cacna2d2, cacna2d3, ClCn1, ClCn2, ClCn3, ClCn4, ClCn5, ClCn6, ClCn7, ClCK1, oder ClCK2.

12. Transgener Ciliat nach Anspruch 1, wobei:
a) Adenin- und Thyminnucleotide mindestens 55 % der Nucleotide der codierenden Sequenz des Transgens, das für den spannungsabhängigen Ionenkanal eines Säugers codiert, umfassen; oder
b) mindestens 50 % der Codons der codierenden Sequenz des Transgens, das für den spannungsabhängigen Ionenkanal eines Säugers codiert, aus den bevorzugten Codons für die Expression in dem Ciliaten gewählt sind.

13. Verfahren zum Produzieren eines rekombinanten spannungsabhängigen Ionenkanals eines Säugers in einem transgenen Ciliaten, umfassend:
Bereitstellen des transgenen Ciliaten, wobei der transgene Ciliat ein Transgen umfasst, das für einen spannungsabhängigen Ionenkanal eines Säugers codiert, der funktionsfähig mit regulatorischen Sequenzen verbunden ist, sodass der Ciliat das spannungsabhängige Ionenkanalprotein exprimiert, wobei sich das Ruhemembranpotential des Ciliaten von dem Ruhemembranpotential einer gesunden nativen Zelle, in der das spannungsabhängige Ionenkanalprotein exprimiert wird, um mindestens 10 mV unterscheidet, wobei das Ruhemembranpotential des Ciliaten weniger polarisiert ist als das der gesunden nativen Zelle, in der das spannungsabhängige Ionenkanalprotein exprimiert wird, wobei das Transgen innerhalb eines ribosomalen DNA-Vektors (rDNA-Vektors) vorhanden ist; und
Züchten des transgenen Ciliaten unter Bedingungen, die eine Expression des spannungsabhängigen Ionenkanals erlauben oder induzieren.

14. Transgener Ciliat nach Anspruch 1, wobei der Ciliat etwa 9,000 Kopien des rDNA-Vektors umfasst.

## Revendications

1. Cilié transgénique comprenant :
un transgène codant pour un canal ionique sensible à la tension de mammifère relié de manière opérationnelle à des séquences régulatrices de telle sorte que le cilié exprime la protéine du canal ionique sensible à la tension,
dans lequel le potentiel de membrane au repos du cilié est différent du potentiel de membrane au repos d'une cellule native saine dans laquelle la protéine du canal ionique sensible à la tension est exprimée par au moins 10 mV, dans lequel le potentiel de membrane au repos du cilié est moins polarisé que la cellule native saine dans laquelle la protéine du canal ionique sensible à la tension est exprimée, et
dans lequel le transgène est présent dans un vecteur d'ADN ribosomique (ADNr).

2. Cilié transgénique selon la revendication 1, dans lequel le potentiel de membrane au repos du cilié est différent du potentiel de membrane au repos d'une cellule native saine dans laquelle la protéine du canal ionique sensible à la tension est exprimée par au moins 15 mV ; ou dans lequel le potentiel de membrane au repos du cilié est différent du potentiel de membrane au repos d'une cellule native saine dans laquelle la protéine du canal ionique sensible à la tension est exprimée par au moins 20 mV.

3. Cilié transgénique selon la revendication 1, dans lequel le potentiel de membrane au repos du cilié va de -15 mV à -40 mV, ou de -25 mV à -35 mV.

4. Cilié transgénique selon la revendication 1, dans lequel le vecteur d'ADN ribosomique est le pTRAS.

5. Cilié transgénique selon la revendication 1, dans lequel la cellule native est :
(a) une cellule de mammifère choisie dans le groupe constitué par une cellule musculaire squelettique, une cellule musculaire cardiaque, une cellule musculaire lisse, un astrocyte, un neurone, un lymphocyte, une cellule rénale et une cellule ovarienne ; ou
(b) une cellule de mammifère immortalisée choisie dans le groupe constitué par une cellule HEK, une cellule CHO ou une cellule Jurkat.

6. Cilié transgénique selon la revendication 1, dans lequel le cilié produit :
(a) des glycoprotéines y compris des glycanes liés à l'asparagine sans résidus d'acide sialique ; ou
(b) des glycoprotéines qui comprennent au moins un glycane choisi dans le groupe constitué par Mans-, Man₄- et Man₃-GlcNAc₂.

7. Cilié transgénique selon la revendication 1, dans lequel le cilié est une espèce *Tetrahymena,* éventuellement dans lequel l'espèce *Tetrahymena* est le *Tetrahymena thermophila.*

8. Cilié transgénique selon la revendication 1, dans lequel les séquences régulatrices comprennent un promoteur qui est activé par un passage à des conditions de jeune, éventuellement dans lequel le promoteur régule l'expression d'un gène choisi parmi TTHERM_00013600, TTHERM_00013620,
TTHERM_00013640, TTHERM_00024150, TTHERM_00024160, TTHERM_00024230, TTHERM_00671930, TTHERM_ 00735400, TTHERM_00753320, TTHERM_00754720, TTHERM_02255850, TTHERM_00155630, TTHERM_00158010, TTHERM_00805870, TTHERM_00816260, TTHERM_00185390, TTHERM_00833720, TTHERM_00862740, TTHERM_00864910, TTHERM_00864920, TTHERM_00879290,TTHERM_00879300, TTHERM_0081360, TTHERM_00881380, TTHERM_00881390, TTHERM_00881410, TTHERM_00881420, TTHERM_00881440, TTHERM_00881450, TTHERM_00891190, TTHERM_0019330, TTHERM_00193310, TTHERM_02465150, TTHERM_00940290, TTHERM_00227590, TTHERM_00227620, TTHERM_01055590, TTHERM_01060860, TTHERM_01066970, TTHERM_00251250, TTHERM_00251260, TTHERM_00252260, TTHERM_00257060, TTHERM_00257110, TTHERM_00257120, TTHERM_01087790,
TTHERM_01144930, TTHERM_01205390, TTHERM_01220430, TTHERM_01250000, TTHERM_00049210, TTHERM_01349990, TTHERM_01353120, TTHERM_01353130, TTHERM_00338490, TTHERM_00339880, TTHERM_00191020, TTHERM_00191040, TTHERM_00191050, TTHERM_00191060, TTHERM_00129450, TTHRRM_00467230, TTHERM_00525070, TTHERM_00376190, TTHERM_01508150, TTHERM_00418100, TTHERM_00428610, TTHERM_00079390, TTHERM_00449650, TTHERM_00450850, TTHERM_00450940, TTHERM_01658040, TTHERM_00463820, TTHERM_00094270, TTHERM_00532380, TTHERM_00107030, TTHERM_00577290, TTHERM_00113080, TTHERM_00117560, TTHERM_00637310, TTHERM_00637330, TTHERM_01999410, ou TTHERM_00655440.

9. Cilié transgénique selon la revendication 1, dans lequel les séquences régulatrices comprennent un promoteur de métallothionéine inductible, éventuellement dans lequel le promoteur régule l'expression d'un gène choisi parmi MTT1, MTT2, MTT3, MTT4, ou MTT5.

10. Cilié transgénique selon la revendication 8 dans lequel le promoteur est :
(a) endogène aux ciliés ; ou
(b) endogène à *Tetrahymena thermophila.*

11. Cilié transgénique selon la revendication 1, dans lequel le canal ionique sensible à la tension :
(a) comprend une sous-unité α porogène ;
(b) est sensiblement exempt de sous-unités auxiliaires ;
(c) est choisi dans le groupe constitué par les canaux ioniques sensibles à la tension du sodium, du calcium, et du potassium ; ou
(d) est choisi parmi les canaux ioniques sensibles à la tension Nav1, Nav1.2, Nav1.3, Nav1.4, Nav1.5,
Nav1.6, Nav1.7, Nav1.8, Nav1.9, Nav2.1/Nav2.2, Nav3, Kv1.1, Kv1.2, Kv1.3, Kv1.4, Kv1.5, Kv1.6, Kv1.7, Kv1.8, Kv2.1, Kv2.2, Kv3.1, Kv3.2, Kv3.3, Kv3.4, Kv4.1, Kv4.2, Kv4.3, Kv5.1, Kv6.1, Kv6.2, Kv6.3, Kv6.4, Kv7.1, Kv7.2, Kv7.3, Kv7.4, Kv7.5, Kv8.1, Kv8.2, Kv9.1, Kv9.2, Kv9.3, Kv10.1, Kv10,2, Kv11.1, Kv11.1.2, Kv11.3, Kv12.1, Kv12.2, Kv12.3, KvBeta1, KvBeta2, Cav1.1, Cav1.2, Cav1.4, Cav2.1, Cav2.2, Cav2.3, Cav3.1, Cav3.2, Cav3.3, cacnb1, cacnb2, cacnb3, cacnb4, cacng1, cacng2, cacng3, cacng4, cacng5, cacng6, cacng7, cacng8, cacna2d1, cacna2d2, cacna2d3, ClCn1, ClCn2, ClCn3, ClCn4, ClCn5, ClCn6, ClCn7, ClCK1,ou ClCK2.

12. Cilié transgénique selon la revendication 1, dans lequel :
a) les nucléotides d'adénine et de thymine comprennent au moins 55 % des nucléotides de la séquence de codage du transgène codant pour le canal ionique sensible à la tension de mammifère ; ou
b) au moins 50 % des codons de la séquence de codage du transgène codant pour le canal ionique sensible à la tension de mammifère sont choisis parmi les codons préférés pour l'expression chez le cilié.

13. Procédé de production d'un canal ionique recombinant sensible à la tension de mammifère chez un cilié transgénique comprenant :
l'apport du cilié transgénique, le cilié transgénique comprenant un transgène codant pour un canal ionique sensible à la tension de mammifère relié de manière opérationnelle à des séquences régulatrices de telle sorte que le cilié exprime la protéine du canal ionique sensible à la tension, dans lequel le potentiel de membrane au repos du cilié est différent du potentiel de membrane au repos d'une cellule native saine dans laquelle la protéine du canal ionique sensible à la tension est exprimée par au moins 10 mV, dans lequel le potentiel de membrane au repos du cilié est moins polarisé que la cellule native saine dans laquelle la protéine du canal ionique sensible à la tension est exprimée, dans lequel le transgène est présent dans un vecteur d'ADN ribosomique (ADNr) ; et
la mise en culture du cilié transgénique dans des conditions qui permettent ou induisent l'expression du canal ionique sensible à la tension.

14. Cilié transgénique selon la revendication 1, dans lequel le cilié comprend environ 9,000 copies du vecteur d'ADNr.
